# EUROPEAN PATENT APPLICATION

(11) **EP 4 606 816 A1**
(43) Date of publication of application: **27.08.2025**
(21) Application number: 24159714.5
(22) Date of filing: 26.02.2024
(51) Int. Cl.: C07K 14/005, C12N 15/864, C12N 15/90

(54) **METHOD FOR PRODUCTION OF GENE THERAPY VEHICLES IN MAMMALIAN CELLS**

(71) Applicant: Universitat Autónoma De Barcelona (UAB), 08193 Bellaterra (Cerdanyola del Valles) (ES)
(72) Inventor: BOSCH MOLIST, Laia, 08510 Roda de Ter (Barcelona) (ES); CERVERA GRACIA, Laura, 08013 Barcelona (ES); LAVADO GARCIA, Jesús, Kobenhavn 2400 (DK); GODIA CASABLANCAS, Francesc, 08201 Sabadell (Barcelona) (ES); LEON MADRENAS, Xavier, 17220 Sant Feliu de Guixols (Girona) (ES); GARCIA MARTINEZ, Miguel, 08225 Terrassa (Barcelona) (ES)

(57) **Abstract**

The present invention relates to a method for production of viruses, viral derivatives, extracellular vesicles (EVs), such as exosomes or microvesicles, or recombinant viral vectors in a cell line, comprising the step of overexpressing at least one of the following genes E1A, CD63, SMPD3A, RAB32, RAB33, RAB35, VPS37B, SNAP47, MVB12A, MVB12B, CD9, CD81, CD151, RAB27A, RAB25, RAB11A, RAB18, SMPD1, UBT8, SGPP1, SMPD4, SNAPIN, SNAP23, SNAP29, VTI1A, VTI1B, and/or E1B in said cell line as well as to a cell line stably overexpressing at least one of these genes.

## Description

### Technical field

The present invention relates to a method for production of viruses, viral derivatives, extracellular vesicles (EVs), such as exosomes or microvesicles, or recombinant viral vectors in a cell line, comprising the step of overexpressing at least one of the following genes E1A, CD63, SMPD3A, RAB32, RAB33, RAB35, VPS37B, SNAP47, MVB12A, MVB12B, CD9, CD81, CD151, RAB27A, RAB25, RAB11A, RAB18, SMPD1, UBT8, SGPP1, SMPD4, SNAPIN, SNAP23, SNAP29, VTI1A, VTI1B, and/or E1B in said cell line, as well as to a cell line stably overexpressing at least one of these genes.

### Background of the invention

In recent years various gene therapy vehicles have emerged that assist with the targeted delivery of gene therapeutics, such as viruses, viral derivatives, extracellular vesicles (EVs) such as exosomes or microvesicles, or recombinant viral vectors.

Cells release diverse types of membrane vesicles of endosomal and plasma membrane origin called exosomes and microvesicles, respectively, into the extracellular environment. These extracellular vesicles (EVs) represent an important mode of intercellular communication by serving as vehicles for transfer between cells of membrane and cytosolic proteins, lipids, and RNA. Exosomes have been successfully engineered to encapsulate and deliver gene therapy vectors to the desired tissues and cells using their ability to pass the cell membrane barrier and at the same time protect the cargo from degradation.

In recent years the gene therapy field has furthermore seen a wave of drugs based on viral vectors that have gained regulatory approval, three key vector strategies being based on adenoviruses, adeno-associated viruses (AAVs), and lentiviruses.

One specific type of recombinant viral vectors, recombinant AAVs (rAAVs) have emerged as one of the vectors of choice for many gene therapy applications due to its desirable characteristics. rAAV vectors have shown an excellent safety profile, they are poorly immunogenic and have not been associated with any disease known in humans (1).

For the successful development of gene therapy drugs using any of these vehicles for their delivery, one key factor is the possibility to upscale the production of high purity vehicles, however, production of large quantities in high quality and at reasonable costs remains a challenge in the field.

For example, current manufacturing systems of rAAV vectors are labour-intensive, resulting in high production costs that need to be reduced to sustain the rAAV use for human gene therapy (11). Therefore, there is an ongoing significant effort towards the design of simple and efficient processes for scalable rAAV production in accordance with cGMP.

Currently, the most widely used method for rAAV production is transient transfection of mammalian cells (12-14). Recent studies have described the triple transfection method of HEK293 suspension-adapted cells under serum-free conditions using polyethylenimine (PEI) transfection reagent (5,15). WO2020/193698 discloses methods for the production of recombinant viral vectors that are improved via repeated transfection as opposed to the conventional approach with only one transfection round.

It is furthermore known that adenoviral genes E1A, E1A19K, E1B55K, the VA RNAs, E2A, and E4orf612-14 are necessary for rAAV propagation (38). E1A plays a critical role in initiating viral replication by increasing transcription from rep gene promoters p5 and p19 (39), as well as activating early adenovirus promoters. Additionally, E1A is required to drive host cells into the S-phase of the cell cycle for viral DNA replication through cellular pRb binding.

HEK293 cell line, a cell line widely used for production of rAAVs, constitutively expresses E1A and E1B genes from adenovirus (16). Activation of AAV promoters p5 and p19 via E1A increases rep expression initiating the AAV replication process. The three plasmids normally used in the production process carry the necessary components for rAAV assembly. One plasmid encodes rep and cap genes of AAV; one plasmid encodes additional helper genes necessary for rAAV production; one plasmid contains an expression cassette with the gene of interest flanked by two ITRS. Using this production method, rAAV vector particles are assembled and accumulate inside the cells and are typically harvested by cell lysis. Whilst cell lysis can be upscaled, the downside of this method is that it requires extensive purification, as viral vectors must be separated from cell debris by costly chromatography or gradient centrifugation steps (17-20).

Previous studies have shown that only a fraction of rAAV vectors are naturally released into the supernatant fraction of the cell-culture media associated with microvesicles (named exosome [exo]-associated AAV) (21-23). Viral vectors can be purified from the supernatant fraction, which is not containing any substantial amount of cell debris, with a simplified and more cost-effective downstream process protocol (24). Although the exo-AAV pathway remains unknown, the release property has been shown to be serotype-specific (21).

Several studies have shown that exo-AAV outperform conventional AAV vectors in terms of transduction efficiency and have a marked resistance to neutralizing antibodies (25-27). The natural shield of exosomes in exo-AAV also attributes new characteristics such as more efficiency in passing biological barriers, such as the blood-brain barrier or the retina inner membrane (28, 29).

However, the amount of exo-AAV recovered from supernatant remains low and recent studies were focused on increasing rAAV secretion into the cell culture medium. The first bioreactor-scale production of rAAV harvested from cell culture media using suspension HEK293 cell line was reported by Samulski et al. (2016) (5). Continuous rAAV harvesting was achieved by medium replacement at various time points to maximize production. Another technical approach was made by Gruber et al. (2018) in which a CD9-overexpression cell line increased the yield of exo-AAVs (30).

To date no method for enhancing exo-rAAV production in mammalian cells has been described in the prior art that allows on the one hand for high propagation of rAAVs in the producing cells and on the other hand for a high amount of rAAVs to be secreted into the cell culture medium giving the possibility for recovery from the supernatant. Whilst recent studies on increasing rAAV secretion in the production process exist, none of these processes provide a sufficient increase, yet, for the large-scale manufacturing of rAAV vectors. Control of the genes of the production pathway of rAAVs, specifically of E1A, has also not been described to date.

The inventors have therefore herein developed a new method using rAAV release pathway modulation as well as modulation of relevant genes for the rAAV propagation by overexpression of specific identified genes that lead to improved rAAV propagation as well as the increase of the extracellular rAAV titer in comparison to the previously reported prior art methods. This method thus provides for an improvement of large-scale manufacturing of rAAV vectors reducing production costs associated to the purification step due to rAAV recovery from cell supernatant.

### Summary of the invention

The present invention therefore relates in one aspect to a method for the production of viruses, viral derivatives, extracellular vesicles (EVs), such as exosomes or microvesicles, or recombinant viral vectors in a cell line, comprising the step of overexpressing at least one of the following genes E1A, CD63, SMPD3A, RAB32, RAB33, RAB35, VPS37B, SNAP47, MVB12A, MVB12B, CD9, CD81, CD151, RAB27A, RAB25, RAB11A, RAB18, SMPD1, UBT8, SGPP1, SMPD4, SNAPIN, SNAP23, SNAP29, VTI1A, VTI1B, and/or E1B in said cell line.

In one embodiment the method of present invention comprises the step of overexpressing at least E1A in said cell line.

In one embodiment the method of present invention comprises the step of overexpressing at least E1A and at least one of the following genes CD63, SMPD3A, RAB32, RAB33, RAB35, VPS37B, SNAP47, MVB12A, MVB12B, CD9, CD81, CD151, RAB27A, RAB25, RAB11A, RAB18, SMPD1, UBT8, SGPP1, SMPD4, SNAPIN, SNAP23, SNAP29, VTI1A, VTI1B, and/or E1B in said cell line.

In one embodiment the method of present invention comprises the step of overexpressing at least one of the following genes E1A, CD63, SMPD3A, Rab32, Rab33, Rab35, VPS37B and/or SNAP47 in said cell line.

In one embodiment of the method of present invention recombinant viral vectors are produced, wherein the recombinant viral vectors are selected from the group consisting of retroviral vectors, adenoviral vectors, lentiviral vectors and adeno-associated viral vectors (rAAV).

In one embodiment the rAAV is selected from AAV serotypes 1 (AAV1), AAV2, AAV3, AAV4, AAV5, AAV6, AAV7, AAV8, AAV9, AAV10, AAV11, AAVDJ, AAVDJ8, AAVrh10, hybrids of two or more different of said serotypes, and said serotypes having mutations that alter the tropism of the AAV serotype.

In one embodiment the rAAV is selected from serotypes AAV1, AAV2, AAV6, AAV8 and AAV9.

In a further embodiment the cell line is selected from HEK293, HEK293T, HEK293FT, CHO, HT1080, HeLa, Vero, Sf9, CAP cells, AGE1.hn, PER.C6, NSO1, COS-7, BHK, CV1, MDCK, BRL3A, W138, HeLa, A549 and HepG2 cells.

In one embodiment the method of present invention comprises the step of overexpressing E1A.

In one embodiment the method of present invention comprises the step of overexpressing SNAP47 in said cell line. In a preferred embodiment the method of present invention comprises the step of overexpressing only SNAP47.

In one embodiment the method of present invention comprises the step of overexpressing VPS37B in said cell line. In a preferred embodiment the method of present invention comprises the step of overexpressing only VPS37B.

In another preferred embodiment the method of present invention comprises the step of overexpressing SNAP47 and SMPD3 in said cell line.

In a further preferred embodiment, the method of present invention comprises the step of overexpressing CD63 in combination with VPS37B or SNAP47 or SMPD3in said cell line.

In yet another preferred embodiment the method of present invention comprises the step of overexpressing CD63, SMPD3A, Rab32, VPS37B, SNAP47 and E1A. In a more preferred embodiment, the method of present invention comprises the step of overexpressing CD63, SMPD3A, Rab32, VPS37B, SNAP47 constitutively and E1A inducibly.

In a further embodiment said method comprises a step of transfecting said cell line with a nucleic acid construct, preferably a plasmid, for the overexpression of the respective gene(s).

In another embodiment said cell line is a cell line that stably overexpresses one or more of the following genes E1A, CD63, SMPD3A, RAB32, RAB33, RAB35, VPS37B, SNAP47, MVB12A, MVB12B, CD9, CD81, CD151, RAB27A, RAB25, RAB11A, RAB18, SMPD1, UBT8, SGPP1, SMPD4, SNAPIN, SNAP23, SNAP29, VTI1A, VTI1B, and/or E1B in said cell line.

In a preferred embodiment said cell line is a cell line that stably overexpresses E1A.

In another embodiment said cell line is a cell line that stably overexpresses E1A and at least one or more of the following genes CD63, SMPD3A, Rab32, Rab33, Rab35, VPS37B, SNAP47, MVB12A, MVB12B, CD9, CD81, CD151, RAB27A, RAB25, RAB11A, RAB18, SMPD1, UBT8, SGPP1, SMPD4, SNAPIN, SNAP23, SNAP29, VTI1A, VTI1B, and/or E1B.

In one embodiment of the method of present invention the method further comprises the steps of:
a. transfecting a cell culture with at least two plasmid vectors, said plasmid vectors comprising a heterologous nucleotide sequence and replication and packaging gene sequences;
b. culturing said cells in a cell culture medium and under conditions allowing viral particle replication and packaging;
c. recovering the viral vectors produced in step b and retaining the cells in the cell culture under conditions allowing further division and growth.

In one embodiment the method further comprising the steps of:
d. re-transfecting the cells according to step c with the plasmid vectors according to step a; and/or
e. repeating steps b to c.

In one embodiment of the method of present invention the cell culture medium of the cell culture is exchanged at least once during the method of production.

In one preferred embodiment the method of present invention comprises the step of overexpressing E1A and a step of exchanging the cell culture medium of the cell culture at least once during the method of production.

In one embodiment of the method the produced viral vectors are secreted to the supernatant of the cell culture in step b.

In one embodiment the cell media of the cell culture is exchanged before step d, preferably wherein cell media exchange is performed by perfusion.

In one embodiment the viral vector is a recombinant AAV and said at least two plasmid vectors comprise a heterologous nucleotide sequence flanked by ITRs, AAV rep and AAV cap gene sequences, and adenovirus helper functions sequences.

In a further aspect the present invention relates to a cell line stably overexpressing at least one of the following genes E1A, CD63, SMPD3A, RAB32, RAB33, RAB35, VPS37B, SNAP47, MVB12A, MVB12B, CD9, CD81, CD151, RAB27A, RAB25, RAB11A, RAB18, SMPD1, UBT8, SGPP1, SMPD4, SNAPIN, SNAP23, SNAP29, VTI1A, VTI1B, and/or E1B in said cell line.

In one embodiment the cell line stably overexpresses E1A.

In one embodiment the cell line stably overexpresses E1A and at least one of the following genes CD63, SMPD3A, RAB32, RAB33, RAB35, VPS37B, SNAP47, MVB12A, MVB12B, CD9, CD81, CD151, RAB27A, RAB25, RAB11A, RAB18, SMPD1, UBT8, SGPP1, SMPD4, SNAPIN, SNAP23, SNAP29, VTI1A, VTI1B, and/or E1B in said cell line.

In a preferred embodiment the cell line stably overexpresses E1A and at least one of the following genes CD63, SMPD3A, Rab32, Rab33, Rab35, VPS37B and/or SNAP47.

In another aspect the present invention relates to the use of the cell line as described herein for the production of viruses, viral derivatives, extracellular vesicles (EVs), such as exosomes or microvesicles, or recombinant viral vectors.

### Brief description of the figures

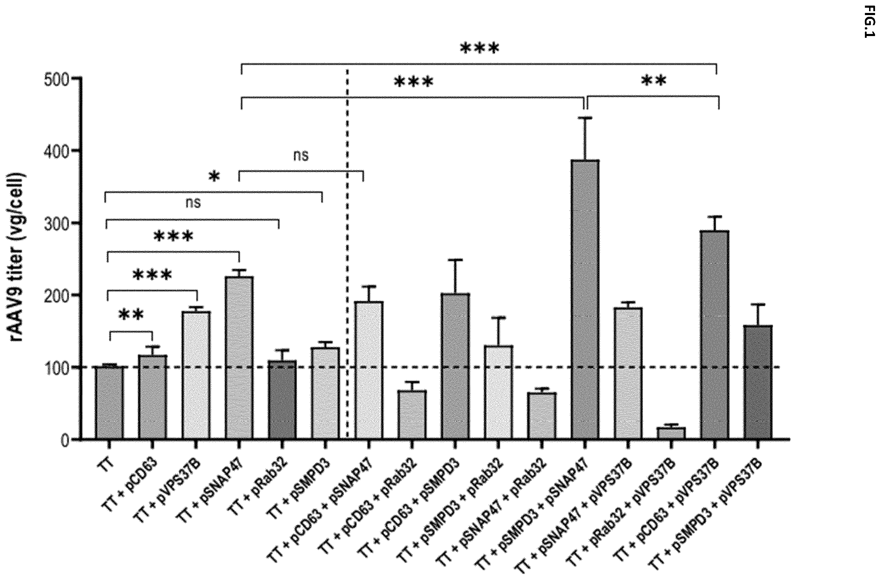

Figure 1: Characterization of rAAV9 titer at cell supernatant expressed as log₂FC (fold change) compared to standard triple transfection. Cells were transfected with standard TT plasmids adding a different test plasmid in each condition as described. Characterization of biological triplicates were measured in triplicate. Data was analyzed by ANOVA test for significant difference *p<0.05; **p<0.01; ***p<0.001; error bars indicate the SD. TT, triple transfection plasmids.

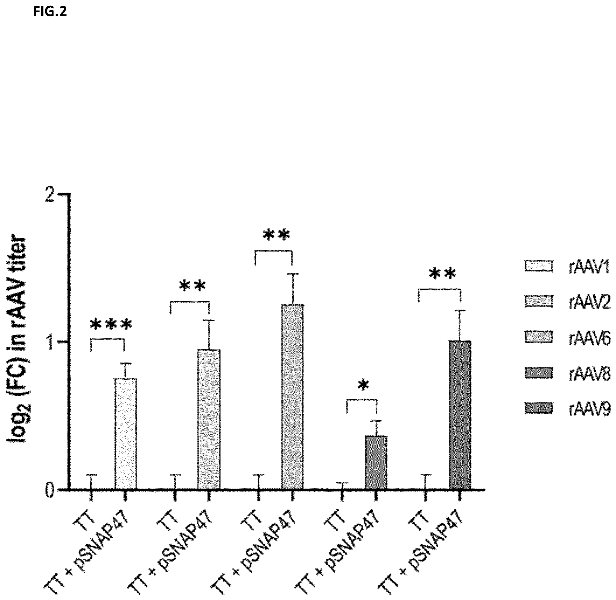

Figure 2: Characterization of rAAV titer at cell supernatant expressed as log₂FC (fold change) compared to standard triple transfection. (A) Overexpression of SNAP47 could outperform standard triple transfection in rAAV harvested at cell supernatant in different serotypes. Biological triplicates were measured in triplicates. Data was analyzed by ANOVA test for significant difference *p<0.05; **p<0.01; ***p<0.001; error bars indicate the SD. Error bars indicate SD. TT, triple transfection plasmids.

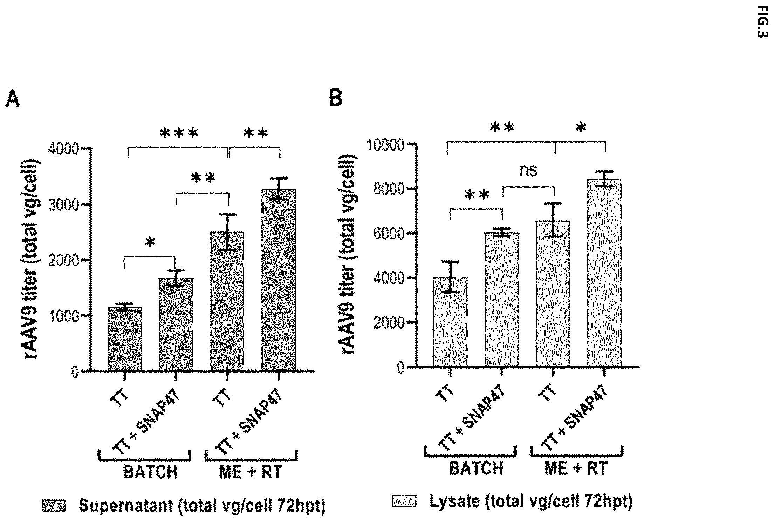

Figure 3: Characterization of rAAV accumulated titer in the form of vg/cell at 72hpt. (A) rAAV harvested from cell supernatant. (B) rAAV harvested from cell lysate. ME was performed at 48hpt. Biological triplicates were measured in triplicate. Data was analyzed by multiple t test for significant difference *p<0.05; **p<0.01; ***p<0.001; error bars indicate the SD. Error bars indicate SD. Vg, vector genomes; TT, triple transfection. ME, medium exchange. RT, re-transfection.

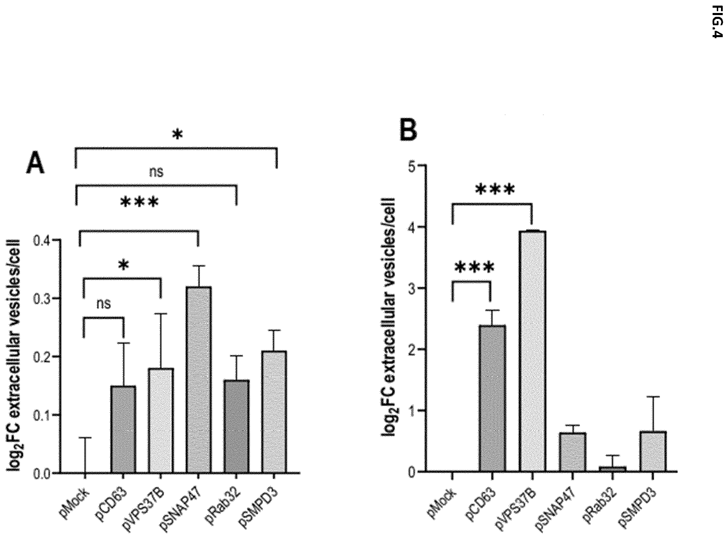

Figure 4: Characterization of extracellular vesicles at cell supernatant in comparison to pMock transfection. (A) Overexpression of individual plasmids in HEK293 cells shows that SNAP47 overexpression has the highest impact in extracellular vesicles production. Medium size of extracellular vesicles produced and modal size. (B) Overexpression of individual plasmids in CHO cells shows that VPS37B overexpression has the highest impact in extracellular vesicles production. Biological triplicates were measured in triplicate. Data was analyzed by ANOVA test for significant difference *p<0.05; **p<0.01; ***p<0.001; error bars indicate the SD.

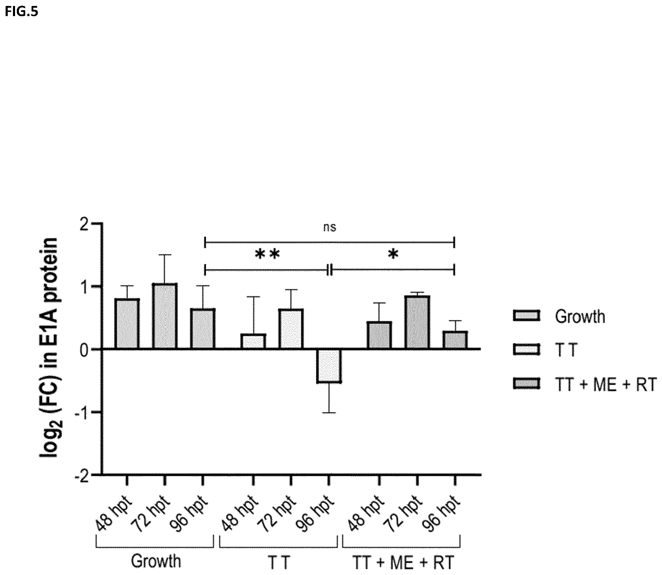

Figure 5: Proteomic data. Bar plot representation of the average Zq values of E1A protein (P03254). E1A protein decreased at 96hpt in TT condition and is reconstituted at 96hpt at EGE condition upon ME and RT. ME was performed at 48hpt. Biological triplicates were measured in triplicates. Data was analyzed by Ordinary ONE way ANOVA *p<0.05;**p<0.01; ***p<0.001; error bars indicate the SD.

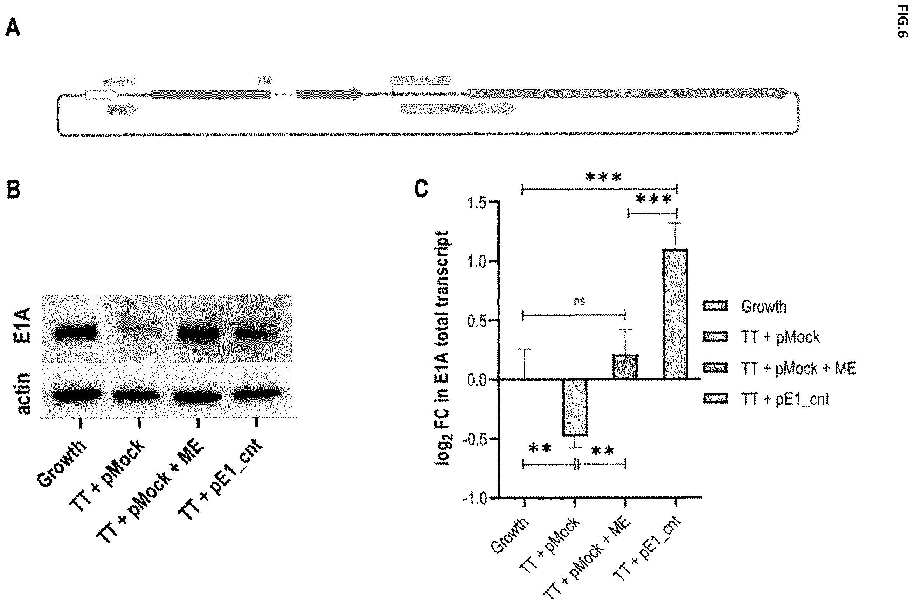

Figure 6: Characterization of E1A protein and mRNA expression at cell pellet 96hpt for rAAV1 production. (A) Schematic representation of pE1_cnt plasmid showing coding region for E1A and E1B. (B) Western Blot of E1A and actin protein expression 96hpt. We observe a clear decrease in E1A protein expression under TT condition that is restored upon ME. (C) Total RNA transcript of E1A shows that upon pE1_cnt plasmid, E1A expression can be overexpressed. ME was performed at 48hpt. Data was analyzed by ANOVA analysis for significant difference *p<0.05; **p<0.01; ***p<0.001; error bars indicate the SD. TT, triple transfection. ME, medium exchange.

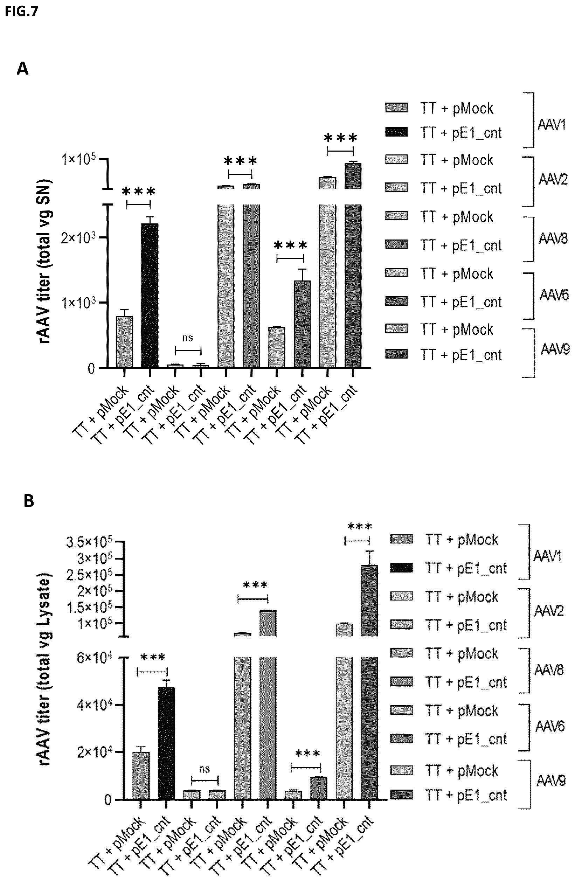

Figure 7: Characterization of rAAV accumulated titer in total vg at cell supernatant and cell lysate at 72hpt for different serotypes. (A) rAAV harvested from cell lysate. (B) rAAV harvested from cell supernatant. Co-transfection of pE1_cnt plasmid increased rAAV titers in all serotypes tested. ME was performed at 48hpt. Biological triplicates were measured in triplicates. ME was performed at 48hpt. Data was analyzed by ANOVA analysis for significant difference *p<0.05; **p<0.01; ***p<0.001; error bars indicate the SD. TT, triple transfection. ME, medium exchange. Vg; vector genomes.

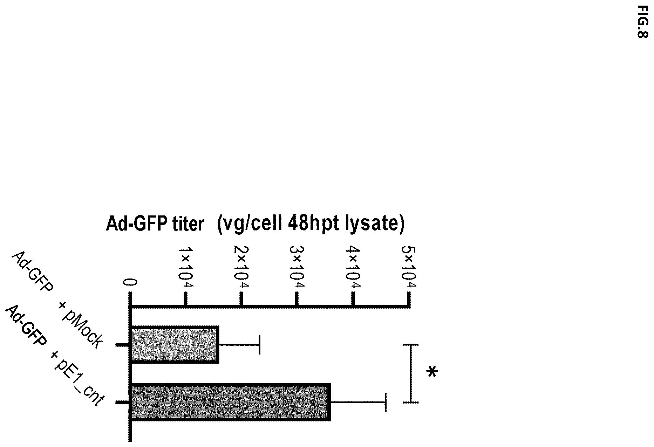

Figure 8: Characterization of AdV5 at cell lysate. Transfection and infection for AdV5 production. Overexpression of E1A could increase total AdV5 production. ME was performed at 24hpt. Biological triplicates were measured in triplicates. Data was analyzed by ANOVA analysis for significant difference *p<0.05; **p<0.01; ***p<0.001; error bars indicate the SD. TT, triple transfection. ME, medium exchange. Vg; vector genomes

### DETAILED DESCRIPTION

The present invention may be understood more readily by reference to the following detailed description of the preferred embodiments of the invention, and to the examples included therein.

As mentioned above, so far, no method for enhancing exo-rAAV production in mammalian cells has been described in the prior art that allows on the one hand for high propagation of rAAVs in the producing cells and on the other hand for a high amount of rAAVs to be secreted into the cell culture medium giving the possibility for recovery from the supernatant. Whilst recent studies on increasing rAAV secretion in the production process exist, none of these processes provide a sufficient increase, yet, for the large-scale manufacturing of rAAV vectors. Control of the genes of the propagation pathway, specifically E1A has also not been described to date.

The inventors have therefore herein developed a new method using rAAV release pathway modulation as well as modulation of relevant genes for the rAAV propagation by overexpression of specific identified genes that lead to improved rAAV propagation as well as the increase of the extracellular rAAV titer in comparison to the previously reported prior art methods. This method thus provides for an improvement of large-scale manufacturing of rAAV vectors reducing production costs associated to the purification step due to rAAV recovery from cell supernatant.

To study the effects on the host cell proteome during rAAV production, and more specifically, metabolic changes occurring during viral vector egress, a proteomic study was conducted under three different conditions. The inventors saw that more than 20 biological process GO terms related to vesicle transport were upregulated upon AAV production.

These processes were not only upregulated in standard production and EGE condition compared to the control non-transfected condition, but they also were upregulated in EGE compared to standard production condition. Biological processes related to lipid biosynthesis were downregulated along cell growth in the control non-transfected condition in agreement to previous reported work (32). Within EGE condition, lipid biosynthesis was significantly upregulated along the time course, increasing at 72 and 96 hpt showing a changing membrane composition with time.

Exosome secretion requires the biogenesis of multivesicular bodies (MVB) and fusion of MVB with the cell membrane. The loading of cargo into MVB has been reported to follow multiple machineries. At least three different routes have been described: the ESCRT pathway, the tetraspanin pathway and a route involving lipids such as sphingomyelin (33).

In this study, proteins from ESCRT-II and ESCRT-III did not show significant differences in EGE conditions, but ESCRT-I protein VPS37B showed a significant increase suggesting a role for ESCRT-I in rAAV secretion.

Tetraspanins have also been described as proteins influencing loading of MVB. However, only the tetraspanin CD63 showed a significant change under EGE condition. CD63 was upregulated compared to non-transfected and to standard production condition. To evaluate the role of CD63 in influencing the secretion of exo-AAVs to the extracellular medium, CD63 expression was modulated and titers of rAAV in the recovered supernatant were monitored. Upon overexpression, extracellular rAAV titers increased significantly (Figure 1).

Another route for loading the MVB with cargo to be later released in the form of exosomes is via ESCRT-independent and tetraspanin-independent pathways like the one triggered by the metabolization of ceramide (35). The expression of sphingomyelinase or sphingomyelin phosphodiesterase enzymes breaking down sphingomyelin to ceramide has been reported to contribute to the pathways sorting cargo into the different routes within the MVB. One of the sphingomyelin phosphodiesterases quantified in the proteomic study, SMPD3 was chosen to be overexpressed and tested for its role in the exo-AAVs production pathway. Upon overexpression of SMPD3, extracellular rAAV titers significantly increased (Figure 1). To test the effect of ceramide, it was added to a control TT (triple transfection) production and to the condition overexpressing SMPD3. Both conditions significantly decreased the extracellular rAAV titers compared to the TT production and to the overexpressed SMPD3 condition (Figure 2).

The next step in exosome generation is the fusion of the MVB to the cell membrane, releasing its cargo to the extracellular medium. Regarding the regulation of this step, the families of Rab and SNAP proteins have been reported to be heavily involved (33,36,37). From more than 46 different quantified Rab proteins; Rab25, Rab32 and Rab18 showed upregulation in EGE condition. From these three Rab proteins, Rab32 was identified with the highest number of proteotypic peptides. Among SNAP proteins, SNAP47 showed the highest upregulation in EGE compared to St. Prod. and non-transfected conditions. Rab32 and SNAP47 were individually overexpressed and SNAP47 only showed an increase in extracellular rAAV titers. The overexpression of Rab32 led to a slight increase in rAAV. However, the significant increase caused by the overexpression of SNAP47 was the highest of the tested conditions (Figure 2). As expected, transfection with a plasmid overexpressing the VPS37B gene resulted in a significant increase in recombinant rAAV titer.

Based on the proteomic study, a series of proteins encompassing the molecular pathways for MVB loading and exosome release were selected to enhance the secretion of exo-AAVs (see example 3). Combinations of CD63, SMPDL3A, Rab32, VPS37B and SNAP47 transient overexpressions were included in the TT process (Figure 1). Upon individual overexpression, VPS37B and SNAP47 showed the highest improvement in secreted rAAV or exo-AAV titer (Figure 1). Combinations of the different gene overexpressions also contribute to increase rAAV titer at the extracellular medium, being the combination of SNAP47 with SMPD3 and CD63 with VPS37B overexpression respectively the ones providing the highest fold change in rAAV titer compared to TT.

The inventors furthermore evaluated the effect of the overexpression of one of the identified genes, SNAP47, on rAAV production capacity at cell supernatant in other exemplary serotypes other than rAAV9 to assess if the effect is serotype specific. Interestingly, they could observe a significant improvement in rAAV1, rAAV2, rAAV6 and rAAV8 titers at cell supernatant in comparison to transient triple transfection with SNAP47 overexpression (Figure 4). No significant differences were observed when a plasmid harboring SNAP47 gene was added to standard triple transfection plasmids in comparison to EGE in cell lysate fractions (Figure 4). Significant improvement was also observed when SNAP47 addition to triple transfection plasmids was transfected under EGE methodology in comparison to EGE with triple transfection plasmids at both cell supernatant and total crude lysate. Overall, this shows the significant improvement added by the co-transfection of pSNAP47 to standard TT plasmids to improve rAAV production.

As explained in more detail in example 5, during the proteomic study, E1A and E1B proteins were identified in all three conditions tested (growth condition (control) and two rAAV production conditions: standard triple transfection (TT) and extended gene expression (EGE)) as they are constitutively expressed in HEK293 cell lines. However, surprisingly, E1A protein was downregulated in triple transfection condition at 96hpt in comparison to growth condition, showing a marked decrease upon triple transfection. At the same time point, E1A expression was restored showing equal levels as in the growth control condition when ME and RT was applied (EGE condition). Thus, ME, RT, or both, are contributing to restore E1A expression to the same level as growth control condition (Figure 5). The inventors thus hypothesized that the decrease observed upon TT may be a limiting factor for rAAV production and assessed how this could be remedied to increase production.

As shown in example 6 when a medium exchange (ME) was performed 48hpt during TT production, similar E1A protein expression levels as in the growth condition were observed (Figure 8). Thus, ME was the factor contributing to E1A protein restoration seen in EGE condition. To investigate if metabolic engineering could restore E1A protein levels during TT, a plasmid overexpressing E1A (pE1_cnt) was cotransfected with TT plasmids performing a quadruple co-transfection. An increase in E1A protein levels was observed (Figure 6). Overall, pE1_cnt supplementation to TT also increased E1A mRNA levels compared to the growth control condition and restored E1A levels that significantly decreased during rAAV production.

After identifying that restoring E1A protein levels through either metabolic engineering or medium exchange during TT production was beneficial, the subsequent effects on the production of rAAV were investigated (example 7). The results demonstrate that E1A restoration through pE1_cnt supplementation is a more effective strategy for enhancing rAAV production in all tested serotypes compared to ME (Figure 7A and B).

The impact of ME and pE1_cnt plasmid addition on other viral particle production, such as adenoviral vector of first generation (Ad-GFP) was also investigated (example 8 and Figure 8). To investigate the impact of E1A overexpression on production, a transfection of either pE1_cnt or pMock plasmid followed by an Ad-GFP infection 4 hpt was conducted. Supplementation with pE1_cnt increased Ad-GFP production by 2-fold compared to the pMock control (Figure 8).

This shows that the effect of E1A overexpression is not limited to rAAV production but is also beneficial to produce other viral particles.

The present invention therefore relates in one aspect to a method for the production of viruses, viral derivatives, extracellular vesicles (EVs), such as exosomes or microvesicles, or recombinant viral vectors in a cell line, comprising the step of overexpressing at least one of the following genes E1A, CD63, SMPD3A, RAB32, RAB33, RAB35, VPS37B, SNAP47, MVB12A, MVB12B, CD9, CD81, CD151, RAB27A, RAB25, RAB11A, RAB18, SMPD1, UBT8, SGPP1, SMPD4, SNAPIN, SNAP23, SNAP29, VTI1A, VTI1B, and/or E1B in said cell line.

In one embodiment the method of present invention comprises the step of overexpressing at least E1A in said cell line.

In one embodiment the method of present invention comprises the step of overexpressing at least E1A and at least one of the following genes CD63, SMPD3A, RAB32, RAB33, RAB35, VPS37B, SNAP47, MVB12A, MVB12B, CD9, CD81, CD151, RAB27A, RAB25, RAB11A, RAB18, SMPD1, UBT8, SGPP1, SMPD4, SNAPIN, SNAP23, SNAP29, VTI1A, VTI1B, and/or E1B in said cell line.

In one embodiment the method of present invention comprises the step of overexpressing at least one of the following genes E1A, CD63, SMPD3A, Rab32, Rab33, Rab35, VPS37B and/or SNAP47 in said cell line.

In a preferred embodiment the method comprises the step of overexpressing at least E1A and at least one of the following genes CD63, SMPD3A, Rab32, Rab33, Rab35, VPS37B, SNAP47 in said cell line.

In one embodiment of the method of present invention the recombinant viral vectors can be selected from the group consisting of retroviral vectors, adenoviral vectors, lentiviral vectors and adeno-associated viral vectors (rAAV).

Adenoviral vectors occupy a special position among the viral systems. They are characterized by a wide host range, by the capability to infect resting cells and by extremely high titers. The infection efficiency related to the necessary amount of nucleic acid exceeds that of all other viral systems and exceeds that of plain DNA 100 000 times (in i.m. application). Types 4 and 7 adenoviruses have been used on a broad scale as live virus vaccines and have a good safety profile. The extremely low integrational tendency of adenoviruses is favorable as an additional safety aspect, since it minimizes the risk of insertion mutagenesis and oncogenic activation. 52 different serotypes of human adenoviruses represent a choice of various viral sheaths with very different tropism. Therefore, they are extremely infectious for the liver or muscles (group C), cells of the central nervous system (members of group D) or cells of the haemopoietic system (group B).

Adeno associated viral (AAV) vectors are widely adopted for *in vivo* organ-directed gene therapy, given their remarkable safety and efficacy profile shown in pre-clinical models and clinical trials. For example, AAV-vector based gene transfer to the liver of clotting factors for the treatment of the coagulation disorder haemophilia is among the most successful applications of gene therapy.

Lentiviral vectors (LV) are attractive vehicles for gene therapy due to their low prevalence of pre-existing immunity against vector components in humans. They can be used as integrative vectors using their intrinsic ability to stably integrate in the genome of target cells, or they can be applied as non-integrative vectors in which case they are integrase deficient LVs.

In one embodiment the rAAV is selected from serotypes selected from serotype AAV1, AAV2, AAV3, AAV4, AAV5, AAV6, AAV7, AAV8, AAV9, AAV10, AAV11 and pseudotyped AAV. In a preferred embodiment the rAAV is selected from serotype AAV1, AAV2, AAV6, AAV8 and AAV9.

Cell lines such as, HEK293, HEK293T, HEK293FT, CHO, HT1080, Vero, HeLa, or Sf9, CAP cells, AGE1.hn, PER.C6, NSO1, COS-7, BHK, CV1, MDCK, BRL3A, W138, HeLa, A549 and HepG2 cells represent important manufacturing platforms in bioengineering. They are widely used to produce gene therapy vehicles. Specifically, HEK293 (human embryonic kidney 293) cells and their derived cell lines provide an attractive heterologous system for the development of vehicle productions. In a further embodiment of the method of present invention the cell line is therefore selected from HEK293, HEK293T, HEK293FT, CHO, HT1080, Vero, HeLa, Sf9, CAP cells, AGE1.hn, PER.C6, NSO1, COS-7, BHK, CV1, MDCK, BRL3A, W138, HeLa, A549 and HepG2 cells.

In a preferred embodiment the cell line is HEK293.

As described above overexpression of certain genes alone or in combination contributes to an increase of rAAV titer at the extracellular medium (Figure 3).

Very high titer was observed when SNAP47 was overexpressed alone or in combination with SMPD3. Thus, in one embodiment of the method of present invention SNAP47 is overexpressed in said cell line. In one preferred embodiment only SNAP47 is overexpressed. In another preferred embodiment SNAP47 and SMPD3 are overexpressed in said cell line.

Very high titer was also observed when VPS37B was overexpressed. Therefore, in one preferred embodiment VPS37B is overexpressed in said cell line. In a further preferred embodiment only VPS37B is overexpressed.

Furthermore, the overexpression of the combination of CD63 with either one of VPS37B or SNAP47 or SMPD3 lead to a high titer. In a further preferred embodiment CD63 in combination with VPS37B or SNAP47 or SMPD3 are thus overexpressed in said cell line.

As described in example 6, restoration of E1A through overexpression is an effective strategy for enhancing rAAV production in different AAV serotypes (Figure 7A and B). In one preferred embodiment of the method of present invention E1A is overexpressed in said cell line. The inventors could show that the levels of E1A decrease during production which can be restored through the overexpression via a plasmid containing E1A gene. In a preferred embodiment the overexpression of E1A is therefore induced to restore the wildtype level of E1A proteins in the cell.

The inventors could furthermore show that combining a step of E1A overexpression and a step of exchanging cell culture medium of the cell culture at least once during the method of production is particularly beneficial for the yield of the present method. The present invention therefore in one specific aspect relates to a method for the production of viruses, viral derivatives, extracellular vesicles (EVs), such as exosomes or microvesicles, or recombinant viral vectors in a cell line, comprising a step of overexpressing E1A and a step of exchanging the cell culture medium of the cell culture at least once during the method of production.

In yet another preferred embodiment of the method of present invention all of the genes CD63, SMPD3A, Rab32, VPS37B, SNAP47 and E1A are overexpressed in the cell line.

It is preferred that CD63, SMPD3A, Rab32, VPS37B, SNAP47 are overexpressed constitutively and E1A is overexpressed inducibly.

In a further embodiment said method comprises a step of transfecting said cell line with a nucleic acid construct, preferably a plasmid, for the overexpression of the respective gene(s).

In another embodiment said cell line is a cell line that stably overexpresses one or more of the following genes E1A, CD63, SMPD3A, RAB32, RAB33, RAB35, VPS37B, SNAP47, MVB12A, MVB12B, CD9, CD81, CD151, RAB27A, RAB25, RAB11A, RAB18, SMPD1, UBT8, SGPP1, SMPD4, SNAPIN, SNAP23, SNAP29, VTI1A, VTI1B, and/or E1B in said cell line.

In one embodiment E1A is overexpressed constitutively or inducibly in a non-E1 transformed cell line. Preferred is inducible expression.

In one embodiment of the method of present invention the method further comprises the steps of:
a. transfecting a cell culture with at least two plasmid vectors, said plasmid vectors comprising a heterologous nucleotide sequence and replication and packaging gene sequences;
b. culturing said cells in a cell culture medium and under conditions allowing viral particle replication and packaging;
c. recovering the viral vectors produced in step b and retaining the cells in the cell culture under conditions allowing further division and growth.

In one embodiment the method further comprising the steps of:
d. re-transfecting the cells according to step c with the plasmid vectors according to step a; and/or
e. repeating steps b to c.

As it is described in the art, genetic material can be introduced into cells using any of a variety of means to transform or transduce such cells. By way of illustration, such techniques include, for example, transfection with bacterial plasmids. Indeed, the plasmid vectors according to step a) of the method of the invention can be introduced into a cell using a variety of transfection techniques. Such transfection methods have been described in the art and include, for example, calcium phosphate co-precipitation, direct microinjection into cultured cells, electroporation, liposome mediated gene transfer, lipid-mediated transfection or nucleic acid delivery using high-velocity microprojectiles. Other suitable transfection media include strontium phosphate, polycationic polymers, e.g., Superfect (QIAGEN(TM)), liposomes, and cationic polymers such as polyethylenimine (PEl).

In one embodiment, the plasmid vectors according to the method of the invention are transfected using PEI. In this case, PEI/DNA complexes are formed by adding PEI to plasmid DNA prior to its addition to the cell culture.

Any of these techniques can be used to introduce one or more exogenous DNA moieties, such as vector constructs, into suitable host cells. Generally, the exogenous DNA must traverse the host cell plasma membrane in order to be exposed to the cell's transcription and replication machinery.

The resulting cell can be transiently transfected with the exogenous nucleic acid molecule, i.e., the exogenous DNA will not be integrated into the genome of a transfected cell, but rather will exist episomally. Alternatively, the resulting cell can be stably transfected, i.e., the nucleic acid molecule will become covalently linked with the host cell genome or will be maintained and replicated as an episomal unit which can be passed on to progeny cells (e.g., capable of extra-chromosomal replication at a sufficient rate).

According to the method of the invention, the plasmid vectors to be transfected comprise a heterologous nucleotide sequence flanked by the ITRs, AAV rep and AAV cap gene sequences, and adenovirus helper functions sequences.

The heterologous polynucleotide is operably linked to its own or to a heterologous promoter, depending for example on the desired level and/or specificity of transcription within the target cell, as is known in the art. Various types of promoters and enhancers are suitable for use in this context. Constitutive promoters provide an ongoing level of gene transcription, and are preferred when it is desired that the therapeutic polynucleotide be expressed on an ongoing basis. Inducible promoters generally exhibit low activity in the absence of the inducer and are up-regulated in the presence of the inducer. They may be preferred when expression is desired only at certain times or at certain locations, or when it is desirable to titrate the level of expression using an inducing agent.

Illustrative examples of promoters are the SV40 late promoter from simian virus 40, the Baculovirus polyhedron enhancer/promoter element, Herpes Simplex Virus thymidine kinase (HSV tk), the immediate early promoter from cytomegalovirus (CMV), the CMV early enhancer/chicken β actin (CAG) promoter and various retroviral promoters including LTR elements. Inducible promoters include heavy metal ion inducible promoters (such as the mouse mammary tumor virus (mMTV) promoter or various growth hormone promoters), and the promoters from T7 phage which are active in the presence of T7 RNA polymerase. A large variety of other promoters are known and generally available in the art, and the sequences for many such promoters are available in sequence databases such as the GenBank database. In a preferred embodiment, the CMV promoter is used.

Where translation is also desired in the intended target cell, the heterologous polynucleotide will preferably also comprise control elements that facilitate translation (such as a ribosome binding site or "RBS" and/or a polyadenylation signal). Accordingly, the heterologous polynucleotide will generally comprise at least one coding region operatively linked to a suitable promoter, and may also comprise, for example, an operatively linked enhancer, ribosome binding site and/or poly-A signal. The heterologous polynucleotide may comprise one coding region, or more than one coding regions under the control of the same or different promoters. The entire unit, containing a combination of control elements and coding or non-coding region, is often referred to as an expression cassette. In a particular embodiment, the heterologous polynucleotide according to the method of the invention contains a poly-A signal.

The heterologous polynucleotide is integrated by recombinant techniques into or preferably in place of the AAV genomic coding region and is generally flanked on either side by AAV inverted terminal repeat (ITR) regions. Alternatively, vector constructs with only one ITR can be employed. In a particular embodiment, said heterologous nucleotide sequence is flanked by two ITRs.

Given the encapsidation size limit of the AAV particles, insertion of a large heterologous polynucleotide into the genome necessitates removal of a portion of the AAV sequence.

Removal of one or more AAV genes is in any case desirable, to reduce the likelihood of generating replication-competent AAV ("RCA"). Accordingly, encoding or promoter sequences for rep, cap, or both, are preferably removed, since the functions provided by these genes can be provided in trans.

In one embodiment, the heterologous nucleotide sequence is flanked by AAV ITRs, and the AAV packaging genes to be provided in trans, are introduced into the host cell in separate vector plasmids.

The rep gene is expressed from two promoters, p5 and p19, and produces four proteins designated Rep78, Rep68, Rep52 and Rep40. Only Rep78 and Rep68 are required for AAV duplex DNA replication, but Rep52 and Rep40 appear to be needed for progeny, single-strand DNA accumulation. Rep68 and Rep78 bind specifically to the hairpin conformation of the AAV ITR and possess several enzyme activities required for resolving replication at the AAV termini. Rep78 and Rep68, also exhibit pleiotropic regulatory activities including positive and negative regulation of AAV genes and expression from some heterologous promoters, as well as inhibitory effects on cell growth.

The cap gene encodes capsid proteins VP1, VP2, and VP3. These proteins share a common overlapping sequence, but VP1 and VP2 contain additional amino terminal sequences transcribed from the p40 promoter by use of alternate initiation codons. All three proteins are required for effective capsid production.

Packaging of an AAV vector into viral particles still relies on the presence of a suitable helper virus for AAV or the provision of helper virus functions. According to the method of the present invention, helper virus function gene sequences are provided in plasmid vectors. The presence of significant quantities of infectious helper virus in a preparation of AAV vectors is problematic in that the preparation is intended for use in human administration.

In a particular embodiment of the method of the invention, transfection is performed using two plasmid vectors wherein one of said plasmid vectors comprises a heterologous nucleotide sequence flanked by ITRs and the second plasmid vector comprises the AAV rep and AAV cap gene sequences and adenovirus helper functions sequences. In a preferred embodiment, said second plasmid vector comprises from 5' to 3' an AAV rep coding region, an AAV cap coding region and a nucleotide sequence comprising an AAV p5 promoter region.

In a preferred embodiment of the method of the invention, transfection is performed using three plasmid vectors, i.e., the host cells are triple-transfected with at least one vector encoding a heterologous nucleotide sequence of interest, at least one vector encoding AAV rep and cap genes, and at least one vector encoding adenoviral accessory functions.

According to step b) of the method of the invention, the transfected cells are thus cultured under conditions allowing viral vector replication and packaging. In a particular embodiment, the cells are cultured under conditions allowing rAAV replication and packaging.

Several criteria influence selection of cells for use in producing rAAV particles as described herein. The more preferred cells and cell lines are those that can be easily grown in culture to facilitate large-scale production of recombinant AAV vector preparations. Where large-scale production is desired, the choice of production method will also influence the selection of the host cell. For example, some production techniques and culture vessels or chambers are designed for growth of adherent or attached cells, whereas others are designed for growth of cells in suspension. In the latter case, the host cell would thus preferably be adapted or adaptable to growth in suspension. According to the present invention, large-scale production of rAAVs is desired.

A variety of cells lines are contemplated for use in the large-scale production of rAAV. Particularly suitable for cell culture of rAAV are Human Embryo Kidney (HEK) 293 cell lines, either adherent or selected for growth in suspension. Examples of other suitable cell lines to produce rAAV include Vero cells, HeLa cells, and CHO cell lines.

As used herein, the term "cell line" refers to a population of cells capable of continuous or prolonged growth and division *in vitro.* Often, cell lines are clonal populations derived from a single progenitor cell. It is known in the art that spontaneous or induced changes can occur in karyotype during storage or transfer of such clonal populations. Therefore, cells derived from the cell line referred to may not be precisely identical to the ancestral cells or cultures. Nevertheless, the term "cell line" includes such variants.

Once a cell line is selected, the cell culture device of choice is seeded with cells at a density suitable to support cell culture. The density of cells used to seed a particular device will depend on the size of the device. A variety of volumes may be used to grow the cells in the culture device of choice. The volume of medium used will vary according to the size of the culture device used to grow the culture cells. Large-scale production methods such as suspension culture may be used. AAV particles are then collected, and isolated from the cells used to prepare them.

Cells are cultured under conditions that are permissive for the AAV replication and packaging of the rAAV vector. Culture time is preferably adjusted to correspond to peak production levels. Preferably, at least 100 viral particles are produced per cell; more preferably at least about 1000 per cell, still more preferably at least about 10,000 per cell. Preferably, at least 0.5 x 10⁶, more preferably at least about 1 x 10⁶, even more preferably at least about 2 x 10⁶ RU/ml AAV vectors are produced per 2 x 10⁵ cells during the culture period.

Various growth media may be used in the disclosed invention to achieve large-scale cell growth and AAV production. The selection of growth medium varies depending on the type of cells being cultured. In a particular embodiment, said cells are mammalian cells. In a preferred embodiment, said cell is Human Embryo Kidney (HEK) 293 cell lines. More preferably, said cells are cells suitable for being grown in suspension.

For the culture of adherent cells, roller bottles (cylindrical tissue culture flasks rotated at a given velocity) are used in order to provide the necessary surface for cell attachment. Alternatively, micro-carriers are also a suitable system to support adherent cell cultures. For the culture of suspension cells, stirred tank bioreactors are the most indicated systems, since they enable to reach high cell density cultures that in turn can provide large amounts of rAAV after purification.

In a particular embodiment of the invention, a rocking-motion-type bioreactor is used. In another particular embodiment a stirred tank bioreactor could also be used. Once the cell culture in the reactor reaches a given point, transfection of cells is performed as mentioned above.

According to the method of the invention, step c) comprises recovering the AAVs produced in step b) and maintaining the cells in the cell culture under conditions allowing further division and growth. In a preferred embodiment of the method, step b) is performed culturing said cell in suspension in agitated liquid medium.

According to a particular embodiment of the method of the invention, in step b) the AAVs produced are secreted to the supernatant of the cell culture. Thus, according to step c) of the method of the invention, the rAAVs are harvested from the supernatant and the cells are kept in the cell culture for further division and growth. The spent media or supernatant is then collected with the rAAVs so produced since, as it is shown in the Examples below, AAVs are secreted to the supernatant of the cell culture without the need of cell lysis.

In a particular embodiment, the cell media of the cell culture is exchanged before re-transfection is performed, i.e., before step d). In a particular embodiment, the cell media exchange is performed by centrifugation. In a preferred embodiment, media exchange is performed by perfusion. In a more preferred embodiment, continuous media exchange is performed by perfusion. More preferably, said culture medium is automatically exchanged by a perfusion system. By this system, the culture is replenished with fresh medium while cell-free supernatant is removed using a cell retention device. This process is preferably performed at a constant harvest flow rate. In this regard, in a preferred embodiment, new, fresh, culture media is added to the retained cells to allow them to further divide and grow. The constant addition of nutrients and removal of toxic metabolites allows perfusion cultures to reach and sustain high cell densities over many weeks.

By performing repeated rounds of transfection, production of rAAVs can be extended over time using the same cell culture.

According to step d) of the method of the invention, re-transfection or repeated rounds of transfection are performed in the cell culture, as opposed to the conventional transient gene expression (TGE) approach which entails a single transfection round without medium exchange.

The re-transfection and recovering steps can be repeated more than one time using the same cell culture. In a particular embodiment of the method of the invention, steps d), b) and c) are repeated at least one more time after recovering step c). In another particular embodiment steps d), b) and c) are repeated at least twice after recovering step c). In a more particular embodiment, steps d), b) and c) are repeated at least three times after recovering step c). In a preferred embodiment, steps d), b) and c) are repeated at least four times after recovering step c). In a more particular embodiment, steps d), b) and c) are repeated between one and three times after recovering step c). In a more particular embodiment, steps d), b) and c) are repeated between one and two times after recovering step c). In a preferred embodiment, the method of the invention includes one repetition of steps d), b) and c) after recovering step c). In a preferred embodiment of the method of the invention, steps d), b) and c) are repeated one more time after recovering step c).

As mentioned before, in a particular embodiment of the method of the invention, continuous media exchange is performed by perfusion. In a preferred embodiment, every time transfection is to be performed, perfusion stands. This allows plasmid vectors to enter the cell. In a more preferred embodiment, perfusion stands for a period of at least 1.5 hours, more preferably, of at least 2 hours, more preferably of at least 3 hours, even more preferably of at least 4 hours. In a preferred embodiment, there is a minimum interval of time between each re-transfection round. In a more preferred embodiment, said re-transfections are performed after an interval of time of at least 36 hours, more preferably, of at least 48 hours.

In another particular embodiment, said method comprises an additional step of lysing the cells in the supernatant after all rounds of re-transfection and recovering are finished.

There are several well-known techniques in the state of the art that can be used for cell disruption or cell lysis. Although freeze-thawing and/or sonication can be used to disrupt the cells, such techniques are not very suitable to large-scale preparations. Mechanical lysis techniques are thus preferable in those regards. Detergents and other chemical agents can also be employed to mediate or facilitate lysis. Treatment of lysates with nucleases (such as benzonase) has been found to be helpful for reducing viscosity and improving filterability. Clarification, e.g., by microfiltration to separate vector from at least some portion of the cellular debris, is also helpful for promoting recovery and purification. In a particular embodiment of the invention, said lysing step according to the method of the invention comprises using a nuclease, more preferably, said nuclease is benzonase.

In a particular embodiment of the method of the invention, the supernatant collected containing the rAAVs of the invention as described above is further processed so that the rAAVs are purified. In this regard, in another particular embodiment, the process is performed in a bioreactor coupled to a cell retention membrane, so that the cells are retained inside the reactor system while the rAAVs are collected via the membrane, in a clean supernatant, free of cellular debris and therefore making purification much easier.

In order to be particularly useful for the production of rAAV for gene therapy, it is most desirable for the techniques to be scalable, i.e., applicable in conjunction with large-scale manufacturing devices and procedures.

By way of illustration, the rAAVs can be loaded on a positively charged anion-exchange column, such as an N-charged amino or imino resin (e.g., POROS or any DEAE, TMAE, tertiary or quaternary amine, or PEI-based resin) or a negatively charged cation-exchange column (such as HS, SP, CM or any sulfo-, phospho- or carboxy-based cationic resin). The column can be washed with a buffer. The column can be eluted with a gradient of increasing NaCl concentration, or a gradient of decreasing pH and fractions can be collected and assayed for the presence of rAAV and/or contaminants.

Other procedures can be used in place of or, preferably, in addition to the above-described anion and cation exchange procedures, based on inter-molecular associations mediated by features other than charge as is known in the art. Such other procedures include intermolecular associations based on ligand-receptor pairs (such as antibody-antigen or lectin-carbohydrate interactions), as well as separations based on other attributes of the molecules, such as molecular sieving chromatography based on size and/or shape.

The pool of rAAV-containing fractions eluted from a column as described above can be concentrated and purified by tangential flow filtration (TFF). The preparation is filtered through a membrane, and the product is retained. The retained material can be diafiltered using the membrane with successive washes of a suitable buffer. The final sample is highly enriched for the product and can be filtered and stored for use.

Transfection with the vector plasmids can occur on the day cell seeding is performed, or may be done on day one, day two, day three, day four, or day five post cell seeding depending on the cell seeding density.

In one embodiment of the invention, the host cells of choice are triple-transfected with at least one vector encoding a heterologous nucleotide sequence of interest, at least one vector encoding AAV rep and cap genes, and at least one vector encoding adenoviral accessory functions. In a particular embodiment, triple-transfection is performed using PEI transfection.

In a further embodiment of the method of present invention the cell culture medium of the cell culture is exchanged at least once during the method of production.

In one embodiment of the method the viral vectors are secreted to the supernatant of the cell culture in step b.

In one embodiment the cell media of the cell culture is exchanged before step d, preferably wherein cell media exchange is performed by perfusion.

AAV vectors can be engineered to carry a heterologous nucleotide sequence of interest (e.g., a selected gene, antisense nucleic acid molecule, ribozyme, or the like) by deleting the internal portion of the AAV genome and inserting the DNA sequence of interest between the ITRs. The ITRs are the only sequences required in cis for replication and packaging of the vector genome containing the heterologous nucleotide sequence of interest. The heterologous nucleotide sequence is also typically linked to a promoter sequence capable of driving gene expression in the target cells under certain conditions. Termination signals, such as polyadenylation sites, are usually included in the vector. The rep and cap AAV gene products provide functions for replication and encapsidation of the vector genome, respectively, and it is sufficient for them to be present in trans.

In one embodiment the viral vector is thus a recombinant AAV and said at least two plasmid vectors comprise a heterologous nucleotide sequence flanked by ITRs, AAV rep and AAV cap gene sequences, and adenovirus helper functions sequences.

In a further aspect the present invention relates to a cell line stably overexpressing at least one of the following genes E1A, CD63, SMPD3A, RAB32, RAB33, RAB35, VPS37B, SNAP47, VPS37B, MVB12A, MVB12B, CD9, CD81, CD151, RAB27A, RAB25, RAB11A, RAB18, SMPD1, UBT8, SGPP1, SMPD4, SNAPIN, SNAP23, SNAP29, VTI1A, VTI1B, and/or E1B in said cell line.

In a preferred embodiment said cell line is a cell line that stably overexpresses E1A.

In another embodiment said cell line is a cell line that stably overexpresses E1A and at least one or more of the following genes CD63, SMPD3A, Rab32, Rab33, Rab35, VPS37B, SNAP47, MVB12A, MVB12B, CD9, CD81, CD151, RAB27A, RAB25, RAB11A, RAB18, SMPD1, UBT8, SGPP1, SMPD4, SNAPIN, SNAP23, SNAP29, VTI1A, VTI1B, and/or E1B.

In another aspect the present invention relates to the use of the cell line as described herein to produce viruses, viral derivatives, extracellular vesicles (EVs), such as exosomes or microvesicles, or recombinant viral vectors.

### Definitions

The use of the word "a" or "an" may mean "one," but it is also consistent with the meaning of "one or more," "at least one," and "one or more than one". The use of the term "another" may also refer to one or more. The use of the term "or" in the claims is used to mean "and/or" unless explicitly indicated to refer to alternatives only or the alternatives are mutually exclusive.

As used herein, words of approximation such as, without limitation, "about", "around", "approximately" refers to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skilled in the art recognize the modified feature as still having the required characteristics and capabilities of the unmodified feature. In general, but subject to the preceding discussion, a numerical value herein that is modified by a word of approximation such as "about" may vary from the stated value by ±1, 2, 3, 4, 5, 6, 7, 8, 9, or 10%. Accordingly, the term "about" may mean the indicated value ± 5% of its value, preferably the indicated value ± 2% of its value, most preferably the term "about" means exactly the indicated value (± 0%).

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. The term "comprises" also encompasses and expressly discloses the terms "consists of" and "consists essentially of". As used herein, the phrase "consisting essentially of" limits the scope of a claim to the specified materials or steps and those that do not materially affect the basic and novel characteristic(s) of the claimed invention. As used herein, the phrase "consisting of" excludes any element, step, or ingredient not specified in the claim except for, e.g., impurities ordinarily associated with the element or limitation.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AB, BBC, AAABCCCC, CBBAAA, CABABB, and so forth. The skilled artisan will understand that typically there is no limit on the number of items or terms in any combination, unless otherwise apparent from the context.

A "vector" as used herein refers to a macromolecule or association of macromolecules that comprises or associates with a polynucleotide and which can be used to mediate delivery of the polynucleotide to a cell. Illustrative vectors include, for example, plasmids, viral vectors, liposomes and other gene delivery vehicles.

The terms "adeno-associated virus", "AAV virus", "AAV virion," "AAV viral particle" and "AAV particle", used as synonyms herein, refer to a viral particle composed of at least one capsid protein of AAV (preferably composed of all capsid proteins of a particular AAV serotype) and an encapsulated polynucleotide corresponding to the AAV genome. The wild-type AAV refers to a virus that belongs to the genus Dependovirus, family Parvoviridae. The wild-type AAV genome is approximately 4.7 Kb in length and consists of a single stranded deoxyribonucleic acid (ssDNA) that can be positive or negative-sensed. The wild-type genome includes inverted terminal repeats (ITR) at both ends of the DNA strand, and three open reading frames (ORFs). The ORF rep encodes for four Rep proteins necessary for AAV lifecycle. The ORF cap contains nucleotide sequences encoding capsid proteins: VP1, VP2 and VP3, which interact to form a capsid of icosahedral symmetry. Finally, the AAP ORF, which overlaps with the Cap ORF, encodes for the AAP protein that appears to promote capsid assembly. If the particle comprises a heterologous polynucleotide (i.e. a polynucleotide different from a wild-type AAV genome, such as a transgene to be delivered to a mammalian cell) flanked by AAV ITRs, then it is typically known as "AAV vector particle" or "AAV viral vector" or "AAV vector" or "recombinant AAV vectors". The invention also encompasses the use of double stranded AAV or self-complimentary AAV, also called dsAAV or scAAV.

The term "adeno-associated virus ITRs" or "AAV ITRs", as used herein, refers to the inverted terminal repeats present at both ends of the DNA strand of the genome of an AAV.

The ITR sequences are required for efficient multiplication of the AAV genome. Another property of these sequences is their ability to form a hairpin. This characteristic contributes to their self-priming, which allows the primase-independent synthesis of the second DNA strand. The ITRs have also been shown to be required for both integration of the wild-type AAV DNA into the host cell genome (e.g. in the human 19 th chromosome for serotype 2 AAV) and rescue from it, as well as for efficient encapsidation of the AAV DNA into a fully assembled, deoxyribonuclease-resistant AAV particle. The ITR sequences are about 145 bp in length. Preferably, the entire sequences of the ITRs are used in the genome of the AAV viral vector, although some degree of minor modification of these sequences is permissible. A wild-type ITR sequence may be altered by insertion, deletion or truncation, as long as the ITR mediates the desired functions, e.g. replication, nicking, virus packaging, integration, and/or provirus rescue. Procedures for modifying these ITR sequences are well known in the art. The ITR may be from any wild-type AAV, including but not limited to serotypes 1, 2, 3, 4, 5, 6, 7, 8, 9, 10, 11 or 12 or any other AAV known or later discovered. The AAV comprises two ITRs, which may be the same or different. Further, the two AAV ITRs can be from the same AAV serotype as the AAV capsid or can be different. In a preferred embodiment, the 5' and 3' AAV ITRs derive from AAV1, AAV2, AAV4, AAV5, AAV7, AAV8 and/or AAV9. Preferably ITRs are from AAV2, AAV8 and/or AAV9 being AAV2 the most preferred. In one embodiment, the AAV2 ITRs are selected to generate a pseudotyped AAV (i.e., an AAV having capsid and ITRs derived from different serotypes).

The expression "recombinant viral genome", as used herein, refers to an AAV genome in which at least one extraneous polynucleotide is inserted into the naturally occurring AAV genome. The genome of the AAV according to the invention typically comprises the cis-acting 5' and 3' inverted terminal repeat sequences (ITRs) and an expression cassette. A "rAAV vector" as used herein refers to an AAV vector comprising a polynucleotide sequence not of AAV origin (i.e., a polynucleotide heterologous to AAV), typically a sequence of interest for the genetic transformation of a cell. In preferred vector constructs of this invention, the heterologous polynucleotide is flanked by two AAV inverted terminal repeat sequences (ITRs).

An "AAV virus" or "AAV viral particle" refers to a viral particle composed of at least one AAV capsid protein (preferably by all of the capsid proteins of a wild-type AAV) and an encapsidated polynucleotide. If the particle comprises a heterologous polynucleotide (i.e., a polynucleotide other than a wild-type AAV genome such as a transgene to be delivered to a mammalian cell), it is typically referred to as a "rAAV vector particle" or simply a "rAAV vector".

"Packaging" refers to a series of intracellular events that result in the assembly of the capsid proteins and encapsidation of the vector genome to form an AAV particle.

AAV "rep" and "cap" genes refer to polynucleotide sequences encoding replication and encapsidation proteins of adeno-associated virus. They have been found in all AAV serotypes examined and are described below and in the prior art. AAV rep and cap are referred to herein as AAV "packaging genes".

The term "CAG promoter" refers to the combination formed by the cytomegalovirus early enhancer element, chicken p-actin promoter and 3' splice sequence derived from the rabbit beta-globin gene (See Alexopoulou A, etal., BMC Cell Biology 2008; 9(2): 1-11, Niwa etal, Gene. 1991 Dec 15;108(2):193-9).

The following examples and figures are provided by way of illustration and are not intended to limit the scope of the present invention.

### EXAMPLES

### MATERIALS AND METHODS

### HEK 293 MAMMALIAN CELL LINE AND CULTURE CONDITIONS

The cell line used in this work is a serum-free suspension-adapted HEK293 cell line (HEK293SF-3F6) kindly provided by Dr. Amine Kamen from McGill University (McGill, Montreal, Canada). Cells were cultured in disposable polycarbonate 125 mL flasks with vent cap (Corning^{®}) at 37°C, 5% of CO2 and 85% RH at 130 rpm in a LT-X Kuhner shaker (LT-X Kuhner, Birsfelden, Switzerland). Cell culture media were HyCell^{™} TransFx-H media (Cytiva, Marlborough, MA, USA) supplemented with 4mM GlutaMAX^{™} (Gibco, Life Technologies, Thermo Fisher Scientific) and 0.1% Pluronic^{™} F-68 non-ionic surfactant (Gibco) and Freestyle^{™} F17 Expression media (Gibco, Life Technologies) supplemented with 8 mM GlutaMAX^{™} (Gibco), 0.1% Pluronic^{™} F-68 Non-ionic surfactant (Gibco) and IGF-1 (91590C, Sigma-Aldrich) at a final concentration of 50 g/L. When required according to planned experiments, culture media was supplemented with 2 mg/L ceramide (860052 P, Avanti, Sigma-Aldrich) dissolved in dimethyl sulfoxide 99% (DMSO; W387520, Sigma-Aldrich). Cell concentration and viability were determined using the NucleoCounter^{®}NC-3000 automatic cell counter (Chemometec, Allerod, Denmark) according to manufacturer's instructions.

### PLASMIDS

Plasmids used for triple transfection were Helper, pGFP _ITR and pRep2Cap9. Plasmids pSNAP47 and pRab32 were generated in house following well-known standard procedures.

### rAAV PRODUCTION BY TRANSIENT TRANSFECTION

Transfections were carried out at a cell density of 2·10⁶ cells/mL using a copy number of 4·10¹⁰ of each plasmid in 6-well plates (Z707759, Sigma-Aldrich). DNA:PEI complexes were formed by adding PE! to plasmid DNA diluted in fresh culture media (10% of the total culture volume to be transfected). Transfection reagent PEIpro^{®} (Polyplus-transfection) was used. Briefly, the corresponding DNA mixture was diluted with supplemented HyCell^{™} media and vortexed for 10 seconds. Then PE! was added in 1:2 (w/w) DNA:PEI ratio and vortexed three times, incubated for 15 min at room temperature and then added to the cell culture (5).

Vector genomes were quantified by qPCR as previously described (Ayuso et al. cited supra) using a set of primers specific to a region within the eGFP gene using iTaq Universal SYBR Green Supermix. Statistical analyses were performed with GraphPad Prism 7.00.

For proteomic analysis, cell culture samples were taken at 48, 72 and 96 hpt by centrifuging the culture at 1000xg for 10min. The supernatant was collected and cellular pellets were stored at -80°C. For rAAV9 production analysis, supernatant was collected 72hpt.

### PROTEIN SAMPLES PREPARATION FOR MASS SPECTROMETRY ANALYSES

Pellets of each tested condition were obtained by centrifugation of 500 L of the cell culture at 1000xg for 10 minutes at 4°C. Pellets were stored at -80°C. Protein extraction was performed using extraction buffer (100 mM Tris-HCl pH=8.8, 2mM EDTA, 4% SDS, 100mM DTT) of which 100 L were added to the cell pellet of each condition. Samples were sonicated for 5 minutes and then boiled for another 5 minutes. Protein extracts from pellets samples were quantified with RC/DC Protein Assay (Bio-Rad, Hercules, CA, USA) and stored in -20 °C until the tryptic digestion process. Protein digestion was performed as previously described [32]. Proteins were digested using sequencing grade trypsin (Promega, Madison, Wl, USA) and the filter-assisted sample preparation technology (FASP, Expedeon, San Diego, CA, USA), and the resulting peptides were subjected to TMT-10 plex labelling (AB Sciex, Framingham, MA, USA), joined and desalted. A total of 150 g from samples of each condition was diluted to a final concentration of 100mM of TEAB labeled with TMT-10 plex according to the manufacturer. Protein samples were labeled by adding 41µL of TMT isobaric tag diluted in anhydrous acetonitrile, followed by a 1h-incubation step at room temperature. To quench the reaction, 5% hydroxyl-amine (8µL per sample) was added, incubated 15 min at room temperature and mixed together followed by addition of TFA 1% to lower pH at 3. TMT-labelled samples were equally mixed. Pooled mix was purified using Oasis HLB C18 column (Waters, MA, USA). TMT-labeled mix was fractionated using High pH reversed-phase peptide fractionation kit (Thermo Scientific, San Jose, CA, USA) according to manufacturer's instructions into 5 fractions for further LC-MS/MS analysis.

### LIQUID CHROMATOGRAPHY TANDEM MASS SPECTROMETRY ANALYSIS

The tryptic peptide mixtures were subjected to LC-MS/MS analysis on a nano-HPLC Easy nLC 1000 liquid chromatograph (Thermo Scientific, San Jose, CA, USA) coupled to a QExactive mass spectrometer (Thermo Scientific, San Jose, CA). Peptides were suspended in 0.1% formic acid, loaded onto a C18 reverse-phase trapping column (Acclaim PepMap100, 75-µm internal diameter, 3-µm particle size and 2-cm length, Thermo Scientific), and separated on an analytical C18 nano-column (EASY-Spray column PepMap RSLC C18, 75-µm internal diameter, 3-mm particle size and 50-cm length, Thermo Scientific), in a continuous gradient (8-31%B in 240 min, 31-90%B in 2min, 90%B in 7 min, and 2%B in 30min; where buffer A is 0.1% formic acid in HPLC grade H2O and buffer B is 100% ACN, 0.1% formic acid in HPLC grade H2O). Spectra were acquired using full ion scan mode over the mass-to-charge (m/z) range 390-1500, 70,000 FT-resolution was performed on the top 15 ions in each full MS scan using the data-dependent acquisition mode with 45s dynamic exclusion enabled.

### PROTEIN IDENTIFICATION AND QUANTIFICATION

Protein identification was performed over the raw files using the SEQUEST HT algorithm integrated in the Proteome Discoverer 2.1 (Thermo Finnigan). MS/MS scans were matched against a human database (UniProtKB/Swiss-Prot 2019_10 Release). For database searching, parameters were selected as follows: trypsin digestion with 2 maximum missed cleavage allowed, precursor mass tolerance of 800 ppm, fragment mass tolerance of 0.02 Da. TMT-10 plex labeling at N-terminal and lysine (+229.62932 Da) as well as cysteine carbamidomethylation (+57.021 Da) were chosen as static modifications whereas methionine oxidation (+15.994915 Da) was chosen as dynamic modification. The same MS/MS spectra collections were searched against inverted database constructed from the same target database. SEQUEST results were analyzed by the probability ratio method [33]. False discovery rate (FDR) for identified peptides was calculated in the inverted database search results using the refined method [34]. Quantitative information of TMT reporter ions was extracted from MS/MS spectra for relative quantification of protein abundance to characterize dynamic protein expression profiles in the selected conditions.

### STATISTICAL ANALYSIS

For the comparative analysis of the protein abundance changes we applied Weighted Scan-Peptide-Protein (WSPP) statistical workflow [35], using SanXoT package [36]. These standardized variables, (Zq), express the quantitative values in units of standard deviation [38]. For the protein functional analysis, Systems Biology Triangle (SBT) model [39] was used. This algorithm estimates weighted functional category averages (Zc) from the protein values by performing the protein to category integration. After the integration from spectra to peptide and peptide to protein, this integration represents a higher level, from protein to category. The integration allows the detection of changes in functional categories produced by the coordinated behavior of their proteins [37]. Together with each Zq and Zc, the corresponding FDR was calculated. 5% FDR was considered significant. The quantified proteins were functionally annotated using the Gene Ontology database [40], [41]. For further Gene Ontology annotation, DAVID [42], [43] was used to perform functional enrichment analysis. To help analyze and comprehend the data, the online software for reactions, proteins and pathways analysis REACTOME [44] was used.

### PARTICLE SIZE MEASUREMENT

Dynamic light scattering (DLS) experiments were performed using a Zetasizer Nano ZS instrument (Malvern instruments, Malvern, UK) with a He/Ne 633 nm laser at 173°. The hydrodynamic diameter, particle size distribution in volume, derived count rate (dCR) and polydispersity index (PDI) were calculated with cumulative fit correlation at 25°C and 0.887 cP or 2.448 cP for concentrated samples by ultracentrifugation, respectively. Briefly, 50 µL of sample was placed in disposable plastic cuvettes (UV-Cuvette micro, BRAND GMBH, Germany) followed by automated experimental data collection. Technical triplicates with 12 scans of 10 s were performed in each independent measurement. Nanoparticle tracking analysis (NTA) was performed with a NanoSight^{®} LM20 device (NanoSight Ltd., Amesbury, UK) at the Institut de Ciència de Materials de Barcelona (ICMAB, CSIC, Campus UAB), equipped with a neutral density filter for total particle by light scattering. Data were analyzed with NanoSight^{®} NTA 3.2 software. Briefly, samples were injected being previously diluted 1:20 and three independent analyses were carried out. Three video recordings of 60sec length were made for each sample. Subsequently, particles were identified and tracked by their Brownian motion at room temperature. NTA software allowed determining the size and number of extracellular vesicles.

### EXPERIMENTAL DESIGN AND STATISTICAL RATIONALE

For the multiplexed quantitative proteomics experiment based on TMT-10 plex labeling, three conditions were tested in this study: No transfected condition, following the standard transfection protocol for AAV9 batch production previously explained and transfected following the extended gene expression (EGE) protocol characterized by a medium exchange (ME) and retransfection step at 48 hpt for AAV9 production. Samples from each condition were taken at three different time points: 48, 72 and 96 hpt. The TMT-based isobaric labelling quantification was performed with three biological replicates for each condition and time point. All individual values were normalized by the value from a peptide pool from the non-transfected condition at 48hpt which was used as an internal control for the all the statistical analyses.

### Example 1: Rational optimization of rAAV production pathways

To study the effects on the host cell proteome during rAAV production, and more specifically, metabolic changes occurring during viral vector egress, a proteomic study was conducted. Three different conditions were compared; HEK293 cells growing without transfection, standard triple transfection and triple transfection with medium exchange and re-transfection at 48 hours post transfection (hpt) (known as EGE) to produce rAAV. Samples were taken of cell supernatant at different time points (48, 72 and 96hpt) and used for proteomic comparative profiling via LC-MS/MS analysis. Protein identification was performed as Gene ontology terms, revealing affected cellular processes upon medium exchange during rAAV production. Individual proteins were also quantified showing differential protein changes in each condition. Upon proteomic analysis, a total of 9255 proteins were identified and quantified. Proteins present in all three conditions and quantified with more than 1 peptide accounted for 4679 proteins. Based on the increase of exo-AAV generation upon implementation of the EGE protocol, biological processes and proteins that significantly changed in EGE condition were analyzed.

Vesicle transport was thoroughly studied to identify upregulations in the EGE condition pointing towards the pathways involved and influencing exo-AAVs generation. Identification of these pathways may lead to a modulation of rAAV secretion pathway, resulting in increased rAAV titers at supernatant. More than 20 biological process GO terms related to vesicle transport were upregulated upon AAV production. These processes not only were upregulated in TT and EGE condition compared to the control non-transfected condition, but they also were upregulated in EGE compared to TT condition. Biological processes related to lipid biosynthesis were downregulated along cell growth in the control non-transfected condition in agreement to previous reported work (32). Within EGE condition, lipid biosynthesis was significantly upregulated along the time course, increasing at 72 and 96 hpt showing a changing membrane composition along time.

Exosome secretion requires the biogenesis of multivesicular bodies (MVB) and fusion of MVB with the cell membrane. The loading of cargo into MVB has been reported to follow multiple machineries. At least three different routes have been described: the tetraspanin pathway and a route involving lipids such as sphingomyelin (33).

In the study, proteins from the ESCRT pathway, ESCRT-II and ESCRT-III, did not show significant differences in EGE condition. ESCRT-I protein VPS37B showed a significant increase in EGE at 96hpt, thus indicating a role for ESCRT-I on rAAV secretion.

In the study, out of the tetraspanins, only CD63 was observed to report a significant change in EGE condition. In this condition, CD63 was upregulated compared to non-transfected and to standard production condition. To evaluate the role of CD63 in influencing the secretion of exo-AAVs to the extracellular medium, CD63 expression was modulated and titers of rAAV in the recovered supernatant were monitored. Upon overexpression, extracellular rAAV titers increased significantly (Figure 1). Interestingly, co-transfecting a plasmid encoding a shRNA to knock-down CD63 gene expression, the extracellular titers of rAAV were significantly decreased showing that CD63 pathway is needed for rAAV egress.

One of the sphingomyelin phosphodiesterases quantified in the proteomic study, SMPD3 was chosen to be overexpressed and test its role in the exo-AAVs production pathway. Upon overexpression of SMPD3, extracellular rAAV titers significantly increased (Figure 1). To test the effect of ceramide, it was added to a control TT (triple transfection) production and to the condition overexpressing SMPD3. Both conditions significantly decreased the extracellular rAAV titers compared to the TT production and to the overexpressed SMPD3 condition (Figure 1).

From more than 46 different quantified Rab proteins; Rab25, Rab32 and Rab18 showed upregulation in EGE condition. From these three Rab proteins, Rab32 was identified with the highest number of proteotypic peptides. Among SNAP proteins, SNAP47 showed the highest upregulation in EGE compared to St. Prod. and non-transfected conditions. Rab32 and SNAP47 were individually overexpressed and SNAP47 only showed an increase in extracellular rAAV titers. The overexpression of Rab32 led to a slight increase in rAAV. However, the significant increase caused by the overexpression of SNAP47 was the highest of the tested conditions (Figure 1). As expected, transfection with a plasmid overexpressing the VPS37B gene resulted in a significant increase in recombinant rAAV titer.

### Example 2: Differential specific gene expression enhances rAAV titer at cell supernatant

Based on the proteomic study reported in this work, a series of proteins encompassing the molecular pathways for MVB loading and exosome release were selected to enhance the secretion of exo-AAVs. Combinations of CD63, SMPDL3A, Rab32 and SNAP47 transient overexpressions were included in the TT process (Figure 1). Upon individual overexpression, VPS37B and SNAP47 showed the highest improvement in secreted rAAV or exo-AAV titer (Figure 1). Combinations of the different gene overexpressions also contribute to increase rAAV titer at the extracellular medium, being the combination of SNAP47 with SMPD3 and CD63 with VPS37B overexpression respectively the ones providing the highest fold change in rAAV titer compared to TT.

### Example 3: SNAP47 gene overexpression on rAAV production capacity at cell supernatant in serotypes rAAV1, rAAV2, rAAV6 and rAAV8

The positive impact of SNAP47 gene overexpression on rAAV production capacity at cell supernatant was also evaluated for other serotypes other than rAAV9, which is the one used in the proteomic analysis. Interestingly, there is a significant improvement in rAAV1, rAAV2, rAAV6 and rAAV8 titers at cell supernatant in comparison to transient triple transfection (with the addition of pMock plasmid) with SNAP47 overexpression (Figure 3). Finally, the capacity of SNAP47 overexpression in standard transient transfection to achieve similar titers at cell supernatant as when EGE methodology was assessed. Figure 4 shows that no significant differences are observed when plasmid harboring SNAP47 gene is added to standard triple transfection plasmids (with the addition of pMock plasmid) in comparison to EGE in cell lysate fraction Significant improvement was also observed when SNAP47 addition to triple transfection plasmids was transfected under EGE methodology in comparison to EGE with triple transfection plasmids at both cell supernatant and total crude lysate. Overall, this shows the significant improvement added by the co-transfection of pSNAP47 and pVPS37b to standard TT plasmids to improve rAAV production.

### Example 4: Enhancement of exosome particles production in CHO and HEK293 cell lines Differential specific gene expression enhances exosome production harvested from different cell lines at cell supernatant

Based on the proteomic study, a series of proteins encompassing the molecular pathways for ESCRT-I, MVB loading and exosome release were selected to enhance the secretion of exosomes Combinations of CD63, SMPDL3A, Rab32, SNAP47 and VPS37B transient overexpressions (Figure 4). Upon individual overexpression, VPS37B and SNAP47 showed the highest improvement in exosome production both on HEK293 (Figure 4A) and CHO cell line (Figure 4B).

### Example 5: E1A protein level decreases upon rAAV production by transient triple transfection and is restored upon EGE

In the proteomic study, individual proteins were identified and quantified showing differential changes in different time point conditions (48, 72, 96hpt) in three different conditions including a growth condition (maintained as control) and two rAAV production conditions: standard triple transfection (TT) and extended gene expression (EGE). EGE was achieved by performing a medium exchange (ME) and an additional re-transfection (RT) 48hpt. As expected, E1A and E1B proteins were identified in all three conditions as they are constitutively expressed in HEK293 cell lines. Interestingly, E1A protein was downregulated in TT condition at 96hpt in comparison to growth condition, showing a marked decrease upon triple transfection. At the same time point, E1A expression was restored showing equal levels as in the growth control condition when ME and RT was applied (EGE condition). Thus, ME, RT, or both, are contributing to restore E1A expression to the same level as growth control condition (Figure 5).

The role of adenoviral E1A has been extensively identified as a key factor required for rAAV production (23). Thus, we hypothesize that the decrease observed upon TT may be a limiting factor for rAAV production.

### Example 6: Restoration of E1A gene expression levels using medium exchange or metabolic engineering

To investigate the effect of E1A restoration upon TT production, the direct effect of the ME step included in the EGE methodology on the restoration of E1A expression was investigated. Further, the effect of a metabolic gene engineering approach consisting in E1A over expression was assessed. To this end, a pE1_cnt plasmid was constructed encoding the entire E1 region of wtAdV5 with its endogenous promoters (Figure 6A).

Significantly, when ME was performed 48hpt during TT production, similar E1A protein expression levels as in the growth condition were observed (Figure 6B). Thus, ME is the factor contributing to E1A protein restoration seen in EGE condition. To investigate if metabolic engineering could also be beneficial restore E1A protein levels during TT, pE1_cnt was cotransfected with TT plasmids performing a quadruple co-transfection. When E1A protein expression was evaluated in comparison to TT with pMock transfection, an increase in E1A protein levels was observed (Figure 6B).

Examination of E1A transcript levels during rAAV production, revealed that E1A mRNA levels dropped as expected upon TT condition, while when ME is applied, this E1A reduction was lower. This suggests that ME is able to affect both E1A protein and mRNA levels during rAAV production. Furthermore, addition of the pE1_cnt plasmid resulted in a significant restoration of overall E1A levels (Figure 6C). Overall, pE1_cnt supplementation to TT increased E1A mRNA levels compared to the growth control condition and restored E1A levels that significantly decreased during rAAV production.

### Example 7: E1A supplementation to TT increases rAAV titer in different rAAV serotypes

After identifying that restoring E1A protein levels through either metabolic engineering or medium exchange during TT production was beneficial, the subsequent effects on the production of rAAV were investigated. Three different conditions for rAAV production were tested: TT applying ME at 48hpt and addition of either pE1_cnt plasmid or pMock to TT. To assess the effect of pE1_cnt supplementation on multiple rAAV serotypes, rAAV production using a pRepCap plasmid coding for a different capsid serotype on each condition including rAAV1, rAAV6, rAAV8, and rAAV9 was performed (Figure 7). The total number of rAAV particles per cell in the cell supernatant and cell lysate before the application of ME at 48 hpt, as well as 72hpt were quantified. The analysis revealed that upon ME, clear differences, which are serotype-dependent and cell fraction-dependent, were observed. Notably, ME did not increase rAAV production in the cell lysate fraction for AAV9 and AAV1 serotypes (Figure 7A), but it did result in an increase in the cell supernatant fractions (Figure 7B). Conversely, the opposite was observed for rAAV8. Furthermore, in the case of the cell supernatant fraction, rAAV6 did not benefit from ME treatment. Overall, pE1_cnt supplementation outperformed ME in all rAAV serotypes, resulting in higher levels of rAAV production in both cell supernatant and cell lysate fractions compared to the pMock control condition. These results demonstrate that E1A restoration through pE1_cnt supplementation is a more effective strategy for enhancing rAAV production in all tested serotypes compared to ME (Figure 7A and 7B).

### Example 8: E1A supplementation increase other viral particle production

The impact of pE1_cnt plasmid addition on other viral particle production, such as adenoviral vector of first generation, Ad-GFP hereafter was also investigated (Figure 8). To investigate the impact of E1A overexpression on Ad-GFP production, a transfection of either pE1_cnt or pMock plasmid followed by an Ad-GFPinfection 4 hpt was conducted. This time point was selected to avoid any potential interference with the transfection process. Here, supplementation with pE1_cnt increased Ad-GFP production by 2-fold compared to the pMock control (Figure 8).

### References

1. Mingozzi F, High KA. Immune responses to AAV vectors: overcoming barriers to successful gene therapy. Blood. 2013;122(1):23-36. doi:10.1182/BLOOD-2013-01-306647
2. Zhong L, Li B, Mah CS, et al. Next generation of adeno-associated virus 2 vectors: Point mutations in tyrosines lead to high-efficiency transduction at lower doses. 2008. www.pnas.org/cgi/content/full/. Accessed January 12, 2022.
3. Bartel MA, Weinstein JR, Schaffer D V. Directed evolution of novel adeno-associated viruses for therapeutic gene delivery. Gene Ther 2012 196. 2012;19(6):694-700. doi:10.1038/gt.2012.20
4. Cabanes-Creus M, Navarro RG, Zhu E, et al. Novel human liver-tropic AAV variants define transferable domains that markedly enhance the human tropism of AAV7 and AAV8. Mol Ther - Methods Clin Dev. 2022;24:88-101. doi:10.1016/J.OMTM.2021.11.011
5. Grieger JC, Soltys SM, Samulski RJ. Production of recombinant adeno-associated virus vectors using suspension HEK293 cells and continuous harvest of vector from the culture media for GMP FIX and FLT1 clinical vector. Mol Ther. 2016;24(2):287-297. doi:10.1038/mt.2015.187
6. Aucoin MG, Perrier M, Kamen AA. Critical assessment of current adeno-associated viral vector production and quantification methods. Biotechnol Adv. 2008;26(1):73-88. doi:10.1016/J. BIOTECHADV.2007.09.001
7. Sonntag F, Kother K, Schmidt K, et al. The Assembly-Activating Protein Promotes Capsid Assembly of Different Adeno-Associated Virus Serotypes. J Virol. 2011;85(23):12686-12697. doi:10.1128/jvi.05359-11
8. Ferreira V, Petry H, Salmon F. Immune Responses to AAV-Vectors, the Glybera Example from Bench to Bedside. Front Immunol. 2014;5(MAR). doi:10.3389/FIMMU.2014.00082
9. Jacobson SG, Cideciyan A V., Ratnakaram R, et al. Gene therapy for leber congenital amaurosis caused by RPE65 mutations: safety and efficacy in 15 children and adults followed up to 3 years. Arch Ophthalmol (Chicago, III 1960). 2012;130(1):9-24.
   doi:10.1001/ARCHOPHTHALMOL.2011.298
10. Clément N, Grieger JC. Manufacturing of recombinant adeno-associated viral vectors for clinical trials. Mol Ther - Methods Clin Dev. 2016;3(November 2015):16002. doi:10.1038/mtm.2016.2
11. Collins LT, Ponnazhagan S, Curiel DT. Synthetic Biology Design as a Paradigm Shift toward Manufacturing Affordable Adeno-Associated Virus Gene Therapies. ACS Synth Biol. 2023;12(1):17-26. doi:10.1021/acssynbio.2c00589
12. Grimm D, Kern A, Rittner K, Kleinschmidt JA. Novel tools for production and purification of recombinant adenoassociated virus vectors. Hum Gene Ther. 1998;9(18):2745-2760. doi: 10.1089/H U M. 1998.9.18-2745
13. Matsushita T, Elliger S, Elliger C, et al. Adeno-associated virus vectors can be efficiently produced without helper virus. Gene Ther. 1998;5(7):938-945. doi:10.1038/SJ.GT.3300680
14. Xiao X, Li J, Samulski RJ. Production of high-titer recombinant adeno-associated virus vectors in the absence of helper adenovirus. J Virol. 1998;72(3):2224-2232. http://www.ncbi.nlm.nih.gov/pubmed/9499080%0Ahttp://www.pubmedcentral.nih.gov/articl erender.fcgi?artid=PMC109519.
15. Chahal PS, Schulze E, Tran R, Montes J, Kamen AA. Production of adeno-associated virus (AAV) serotypes by transient transfection of HEK293 cell suspension cultures for gene delivery. J Virol Methods. 2014;196:163-173. doi:10.1016/j.jviromet.2013.10.038
16. Graham FL, Smiley J, Russell WC, Nairn R. Characteristics of a human cell line transformed by DNA from human adenovirus type 5. J Gen Virol. 1977;36(1):59-72. doi:10.1099/0022-1317-36-1-59
17. Smith RH, Ding C, Kotin RM. Serum-free production and column purification of adeno-associated virus type 5. J Virol Methods. 2003;114(2):115-124. doi:10.1016/J.JVIROMET. 2003.09.002
18. Strobel B, Miller FD, Rist W, Lamla T. Comparative Analysis of Cesium Chloride-and lodixanol-Based Purification of Recombinant Adeno-Associated Viral Vectors for Preclinical Applications. doi:10.1089/hgtb.2015.051
19. Gao G, Qu G, Burnham MS, et al. Purification of recombinant adeno-associated virus vectors by column chromatography and its performance in vivo. Hum Gene Ther. 2000;11(15):2079-2091. doi:10.1089/104303400750001390
20. Potter M, Lins B, Mietzsch M, et al. A simplified purification protocol for recombinant adeno-associated virus vectors. Mol Ther - Methods Clin Dev. 2014;1:14034. doi:10.1038/MTM.2014.34
21. Vandenberghe LH, Xiao R, Lock M, Lin J, Korn M, Wilson JM. Efficient serotype-dependent release of functional vector into the culture medium during adeno-associated virus manufacturing. Hum Gene Ther. 2010;21(10):1251-1257. doi:10.1089/hum.2010.107
22. Lock M, Alvira M, Vandenberghe LH, et al. Rapid, Simple, and Versatile Manufacturing of Recombinant Adeno-Associated Viral Vectors at Scale. Hum Gene Ther. 2010;21(10):1259. doi:10.1089/HUM.2010.055
23. Maguire CA, Balaj L, Sivaraman S, et al. Microvesicle-associated AAV vector as a novel gene delivery system. Mol Ther. 2012;20(5):960-971. doi:10.1038/mt.2011.303
24. Lock M, Alvira M, Vandenberghe LH, et al. Rapid, simple, and versatile manufacturing of recombinant adeno-associated viral vectors at scale. Hum Gene Ther. 2010;21(10):1259-1271. doi:10.1089/hum.2010.055
25. György B, Fitzpatrick Z, Crommentuijn MHW, Mu D. Naturally enveloped AAC vectors for shielding neutralising antibodies and robust gene delivery in vivo. Biomaterials. 2015;35(26):7598-7609. doi:10.1016/j.biomaterials.2014.05.032.Naturally
26. Wassmer SJ, Carvalho LS, György B, Vandenberghe LH, Maguire CA. Exosome-associated AAV2 vector mediates robust gene delivery into the murine retina upon intravitreal injection. Sci Rep. 2017;7(November 2016):1-10. doi:10.1038/srep45329
27. Sancho-Albero M, Medel-Martinez A, Martin-Duque P. Use of exosomes as vectors to carry advanced therapies. RSC Adv. 2020;10(40):23975-23987. doi:10.1039/d0ra02414g
28. Hudry E, Martin C, Gandhi S, et al. Exosome-associated AAV vector as a robust and convenient neuroscience tool. Gene Ther. 2016;23(4):380-392. doi:10.1038/gt.2016.11
29. György B, Sage C, Indzhykulian AA, et al. Rescue of Hearing by Gene Delivery to Inner-Ear Hair Cells Using Exosome-Associated AAV. Mol Ther. 2017;25(2):379-391. doi:10.1016/j.ymthe.2016.12.010
30. Schiller LT, Lemus-Diaz N, Rinaldi Ferreira R, Böker KO, Gruber J. Enhanced Production of Exosome-Associated AAV by Overexpression of the Tetraspanin CD9. Mol Ther - Methods Clin Dev. 2018;9(June):278-287. doi:10.1016/j.omtm.2018.03.008
31. Godia-Casablancas, Francesc; Bosch-Tubert, Maria-Fatima; Garcia-Martinez, Miguel; Cervera-Gracia, Laura; Leon-Madrenas, Xavier; Molas-Laplana, Maria; Gutierrez-Granados S. Methods for the manufacture of recombinant viral vectors. (WO2020193698A1)
32. Lavado-Garcia J, Jorge I, Cervera L, Vázquez J, Gòdia F. Multiplexed Quantitative Proteomic Analysis of HEK293 Provides Insights into Molecular Changes Associated with the Cell Density Effect, Transient Transfection, and Virus-Like Particle Production. J Proteome Res. 2020;19(3):1085-1099. doi:10.1021/acs.jproteome.9b00601
33. Colombo M, Raposo G, Théry C. Biogenesis, Secretion, and Intercellular Interactions of Exosomes and Other Extracellular Vesicles. Annu Rev Cell Dev Biol. 2014;30(1):255-289. doi:10.1146/annurev-cellbio-101512-122326
34. Kolmus K, Erdenebat P, Szymanska E, et al. Concurrent depletion of Vps37 proteins evokes ESCRT-I destabilization and profound cellular stress responses. J Cell Sci. 2021;134(1). doi:10.1242/JCS.250951
35. Trajkovic K, Hsu C, Chiantia S, et al. Ceramide Triggers Budding of Exosome Vesicles into Multivesicular Endosomes. Science (80-).2008;319(5867):1244-1247.doi:10.1126/ science.1153124
36. Wei D, Zhan W, Gao Y, et al. RAB31 marks and controls an ESCRT-independent exosome pathway. Cell Res 2020 312. 2020;31(2):157-177. doi:10.1038/s41422-020-00409-1
37. Alenquer M, Amorim MJ, Graham S, Modis Y. Exosome Biogenesis, Regulation, and Function in Viral Infection. Viruses. 2015;7:5066-5083. doi:10.3390/v7092862
38. Samulski RJ, Shenk T. Adenovirus E1B 55-Mr polypeptide facilitates timely cytoplasmic accumulation of adeno-associated virus mRNAs. J Virol. 1988;62(1):206-210. http://www.ncbi.nlm.nih.gov/pubmed/2824848%0Ahttp://www.pubmedcentral.nih.gov/articl erender.fcgi?artid=PMC250520.
39. Tratschin JD, Miller IL, Carter BJ. Genetic analysis of adeno-associated virus: properties of deletion mutants constructed in vitro and evidence for an adeno-associated virus replication function. J Virol. 1984;51(3):611-619. doi:10.1128/jvi.51.3.611-619.1984
40. Löber C, Lenz-Stöppler C, Dobbelstein M. Adenovirus E1-transformed cells grow despite the continuous presence of transcriptionally active p53. J Gen Virol. 2002;83(8):2047-2057. doi:10.1099/0022-1317-83-8-2047
41. Schmidt M, Afione S, Kotin RM. Adeno-Associated Virus Type 2 Rep78 Induces Apoptosis through Caspase Activation Independently of p53. J Virol. 2000;74(20):9441-9450. doi:10.1128/jvi.74.20.9441-9450.2000
42. Marcellus RC, Teodoro JG, Wu T, et al. Adenovirus type 5 early region 4 is responsible for E1A-induced p53-independent apoptosis. J Virol. 1996;70(9):6207-6215. doi:10.1128/jvi.70.9.6207-6215.1996

## Claims

1. A method for the production of viruses, viral derivatives, extracellular vesicles (EVs), such as exosomes or microvesicles, or recombinant viral vectors in a cell line, comprising the step of overexpressing at least one of the following genes E1A, CD63, SMPD3A, RAB32, RAB33, RAB35, VPS37B, SNAP47, MVB12A, MVB12B, CD9, CD81, CD151, RAB27A, RAB25, RAB11A, RAB18, SMPD1, UBT8, SGPP1, SMPD4, SNAPIN, SNAP23, SNAP29, VTI1A, VTI1B, and/or E1B in said cell line, preferably overexpressing at least E1A.

2. The method of claim 1, wherein recombinant viral vectors are produced and wherein said recombinant viral vectors are selected from the group consisting of retroviral vectors, adenoviral vectors, lentiviral vectors and adeno-associated viral vectors (rAAV).

3. The method of any one of the preceding claims, wherein the rAAV is selected from AV serotypes 1 (AAV1), AAV2, AAV3, AAV4, AAV5, AAV6, AAV7, AAV8, AAV9, AAV10, AAV11, AAVDJ, AAVDJ8, AA Vrh1 0, or hybrids of two or more different ones of said serotypes and said serotypes having mutations that alter the tropism of the AAV serotype.

4. The method of any one the preceding claims, wherein the cell line is selected from HEK293, HEK293T, HEK293FT, CHO, HT1080, Vero, Sf9, CAP cells, AGE1.hn, PER.C6, NSO1, COS-7, BHK, CV1, MDCK, BRL3A, W138, HeLa, A549, and HepG2 cells.

5. The method of any one of claims 1 to 4, wherein the method comprises the step of overexpressing E1A.

6. The method of any one of claims 1 to 5, wherein the method comprises the step of
i. overexpressing SNAP47 in said cell line, preferably only overexpressing SNAP47; or
ii. overexpressing VPS37B in said cell line, preferably only overexpressing VPS37B; or
iii. overexpressing SNAP47 and SMPD3 in said cell line; or
iv. overexpressing CD63 in combination with VPS37B or SNAP47 or SMPD3 in said cell line.

7. The method of any one of claims 1 to 6, wherein CD63, SMPD3A, Rab32, VPS37B, SNAP47 and E1A are overexpressed, preferably wherein CD63, SMPD3A, Rab32, VPS37B, SNAP47 are overexpressed constitutively and E1A is overexpressed inducibly.

8. The method of any one of the preceding claims, wherein said method comprises a step of transfecting said cell line with a nucleic acid construct, preferably a plasmid, for the overexpression of the respective gene(s).

9. The method of any one of the preceding claims, wherein said cell line is a cell line that stably overexpresses one or more of the following genes E1A, CD63, SMPD3A, RAB32, RAB33, RAB35, VPS37B, SNAP47, MVB12A, MVB12B, CD9, CD81, CD151, RAB27A, RAB25, RAB11A, RAB18, SMPD1, UBT8, SGPP1, SMPD4, SNAPIN, SNAP23, SNAP29, VTI1A, VTI1B, and/or E1B, preferably wherein the cell line is a cell line that stably overexpresses at least E1A.

10. The method of any of the previous claims, wherein the method further comprises the steps of:
a. transfecting a cell culture with at least two plasmid vectors, said plasmid vectors comprising a heterologous nucleotide sequence and replication and packaging gene sequences;
b. culturing said cells in a cell culture medium under conditions allowing viral particle replication and packaging, preferably wherein in this step the produced viral vectors are secreted to the supernatant of the cell culture;
c. recovering the viral vectors produced in step b and retaining the cells in the cell culture under conditions allowing further division and growth;
and optionally further comprises the steps of
d. re-transfecting the cells according to step c with the plasmid vectors according to step a; and/or
e. repeating steps b to c.

11. The method of claims 9 or 10, wherein the cell culture medium of the cell culture is exchanged at least once during the method of production.

12. The method of claim 11, wherein the cell media of the cell culture is exchanged before step d, preferably wherein cell media exchange is performed by perfusion.

13. The method of any one of the preceding claims, wherein the viral vector is a recombinant AAV and wherein said at least two plasmid vectors comprising a heterologous nucleotide sequence flanked by ITRs, AAV rep and AAV cap gene sequences, and adenovirus helper functions sequences.

14. A cell line stably overexpressing at least one of the following genes E1A, CD63, SMPD3A, RAB32, RAB33, RAB35, VPS37B, SNAP47, MVB12A, MVB12B, CD9, CD81, CD151, RAB27A, RAB25, RAB11A, RAB18, SMPD1, UBT8, SGPP1, SMPD4, SNAPIN, SNAP23, SNAP29, VTI1A, VTI1B, and/or E1B in said cell line, preferably stably overexpressing E1A.

15. Use of the cell line according to claim 14 for the production of viruses, viral derivatives, exosomes, extracellular vesicles (EVs), or recombinant viral vectors.
